# EUROPEAN PATENT SPECIFICATION

(11) **EP 3 350 318 B1**
(45) Date of publication and mention of the grant of the patent: **13.04.2022**
(21) Application number: 16847253.8
(22) Date of filing: 14.09.2016
(51) Int. Cl.: C12N 5/0784, C12N 5/02, C07K 14/535, C07K 14/54, C07K 14/57, C07K 14/525, A61K 35/15, G01N 33/50, G01N 33/569, G01N 33/68

(54) **METHODS RELATING TO ACTIVATED DENDRITIC CELL COMPOSITIONS FOR SUBJECTS WITH ADVANCED CANCERS**
VERFAHREN IM ZUSAMMENHANG MIT AKTIVIERTEN DENDRITISCHEN ZELLZUSAMMENSETZUNGEN FÜR PERSONEN MIT FORTGESCHRITTENEN KREBSERKRANKUNGEN
PROCÉDÉS RELATIFS À DES COMPOSITIONS DE CELLULES DENDRITIQUES ACTIVÉES POUR DES SUJETS ATTEINTS DE CANCERS AVANCÉS

(30) Priority: 15.09.2015 US 201562219058 P
(43) Date of publication of application: 25.07.2018
(60) Divisional application: 21189488.6
(73) Proprietor: NorthWest Biotherapeutics, Inc., Bethesda, MD 20814 (US)
(72) Inventor: BOSCH, Marnix, L., Clyde Hill, WA 98004 (US)
(74) Representative: MacLean, Martin Robert
(86) International application number: PCT/US2016/051781
(87) International publication number: WO 2017/048875

(56) References cited:
- WO-A1-2004/039968
- WO-A1-2016/048872
- US-A1- 2010 330 057
- STRIOGA MARIUS M ET AL: "Therapeutic Dendritic Cell-Based Cancer Vaccines: The State of the Art", CRITICAL REVIEWS IN IMMUNOLOGY, CRC PRESS, INC, US, vol. 33, no. 6, 1 January 2013 (2013-01-01), pages 489-547, XP009178543, ISSN: 1040-8401, DOI: 10.1615/CRITREVIMMUNOL.2013008033
- EUI-HONG BYUN ET AL: "Rv0315, a novel immunostimulatory antigen of, activates dendritic cells and drives Th1 immune responses", JOURNAL OF MOLECULAR MEDICINE, SPRINGER, BERLIN, DE, vol. 90, no. 3, 13 October 2011 (2011-10-13), pages 285-298, XP035024440, ISSN: 1432-1440, DOI: 10.1007/S00109-011-0819-2
- DATABASE BIOSIS [Online] BIOSCIENCES INFORMATION SERVICE, PHILADELPHIA, PA, US; March 2014 (2014-03), YIN ZHILIN ET AL: "Antitumor efficacy of argon-helium cryoablation-generated dendritic cell vaccine in glioma", XP002792224, Database accession no. PREV201400280598 & NEUROREPORT, vol. 25, no. 4, March 2014 (2014-03), pages 199-204, ISSN: 0959-4965(print), DOI: 10.1097/WNR.0000000000000045
- ALINE ZIMMER ET AL: "A regulatory dendritic cell signature correlates with the clinical efficacy of allergen-specific sublingual immunotherapy", JOURNAL OF ALLERGY AND CLINICAL IMMUNOLOGY, vol. 129, no. 4, 23 March 2012 (2012-03-23), pages 1020-1030, XP055536213, AMSTERDAM, NL ISSN: 0091-6749, DOI: 10.1016/j.jaci.2012.02.014
- MITSUHASHI MASATO ET AL: "Discovery of Unique Biomarkers Which Predict Clinical Responses to Dendritic Cell Vaccine", JOURNAL OF IMMUNOTHERAPY, LIPPINCOTT WILLIAMS & WILKINS, US , vol. 35, no. 9 31 October 2012 (2012-10-31), page 743, XP009516361, ISSN: 1524-9557 Retrieved from the Internet: URL:http://journals.lww.com/immunotherapy- journal/pages/default.aspx
- HELLMAN ET AL: "Early Activation Markers of Human Peripheral Dendritic Cells", HUMAN IMMUNOLOGY, NEW YORK, NY, US, vol. 68, no. 5, 24 April 2007 (2007-04-24) , pages 324-333, XP022042793, ISSN: 0198-8859, DOI: 10.1016/J.HUMIMM.2007.01.018
- JIN PING ET AL: "Molecular signatures of maturing dendritic cells: implications for testing the quality of dendritic cell therapies", JOURNAL OF TRANSLATIONAL MEDICINE, BIOMED CENTRAL, vol. 8, no. 1, 15 January 2010 (2010-01-15), page 4, XP021068349, ISSN: 1479-5876
- BUTTERFIELD LISA H ET AL: "Development of a potency assay for human dendritic cells: IL-12p70 production", JOURNAL OF IMMUNOTHERAPY, LIPPINCOTT WILLIAMS & WILKINS, US, vol. 31, no. 1, 31 December 2007 (2007-12-31), pages 89-100, XP009516379, ISSN: 1524-9557, DOI: 10.1097/CJI.0B013E318158FCE0
- CHERYL L-L CHIANG ET AL: "Optimizing parameters for clinical-scale production of high IL-12 secreting dendritic cells pulsed with oxidized whole tumor cell lysate", JOURNAL OF TRANSLATIONAL MEDICINE, BIOMED CENTRAL, vol. 9, no. 1, 14 November 2011 (2011-11-14), page 198, XP021130928, ISSN: 1479-5876, DOI: 10.1186/1479-5876-9-198
- DATABASE BIOSIS [Online] BIOSCIENCES INFORMATION SERVICE, PHILADELPHIA, PA, US; November 2008 (2008-11), HEALEY DON G ET AL: "IL-12 as an Immunopotency Marker for Fully Autologous Dendritic Cell Immunotherapeutics", XP002794707, Database accession no. PREV200900064535 & JOURNAL OF IMMUNOTHERAPY, vol. 31, no. 9, November 2008 (2008-11), page 936, 23RD ANNUAL MEETING OF THE INTERNATIONAL-SOCIETY-FOR-BIOLOGICAL-THERA PY-OF-CANCER; SAN DIEGO, CA, USA; 20081030, ISSN: 1524-9557
- DATTA, J ET AL.: 'Optimizing Dendritic Cell -Based Approaches for Cancer Immunotherapy.' THE YALE JOURNAL OF BIOLOGY AND MEDICINE. vol. 87, no. 4, 12 December 2014, pages 491 - 518, XP055371523
- HAEGEL-KRONENBERGER, H ET AL.: 'Adhesive and/or Signaling Functions of CD 44 Isoforms in Human Dendritic Cells.' THE JOURNAL OF IMMUNOLOGY vol. 161, no. 8, 15 October 1998, pages 3902 - 3911, XP055371525

## Description

### BACKGROUND

Patients with unresectable, locally advanced, or metastatic solid tumors have a poor prognosis and few therapeutic options, especially after having failed standard therapies. Amato, Semin. Oncol. 27:177-186, 2000; Bramwell et al., Cochrane Database Syst. Rev. 3:Cd003293, 2003; Klelger et al., Ann. Oncol. 25:1260-1270, 2014). Recently there have been several promising advances in immune cancer therapies (Ito et al., Biomed. Res. Int. 2015:605478, 2015; West, JAMA Oncol. 1:115, 2015); however, to mount an effective immune response against cancer, the immune system must first be primed to attack cancer cells. (Melero et al., Nat. Rev. Cancer 15:457-472, 2015). Specifically, tumor-specific antigens must be presented to naive T cells by antigen presenting cells, which in turn induce T cell differentiation into activated cytotoxic T cells (CTLs). (Ito et al., Biomed. Res. Int. 2015:605478, 2015; MacKeon et al., Front. Immunol. 6:243, 2015).

Dendritic cells (DCs) are proficient in initiating adaptive immune responses, through the uptake and subsequent presentation to the immune system of antigenic compounds. DCs stimulate both B cells and T cells, and generate costimulatory molecules, such as cytokines, to drive CTL expansion. (Banchereau et al., Nature 392:245-252, 1998). Given the ability of DCs to induce a broad immune response, DC-based immunotherapy research has grown rapidly in recent years. DC-based cancer vaccine clinical trials have shown various degrees of promise, and several products are currently in late-stage clinical trials. (Anguille et al., Pharmacol. Rev. 67:731-753, 2015). The various DCs found in blood are known for their efficient antigen cross-presentation and their ability to effectively migrate to draining lymph nodes. However, DCs compose less than 1% of peripheral blood mononuclear cells, which means that there is insufficient cellular material to generate a composition for initiating and maintaining a tumor specific immune. (MacKeon, Front. Immunol. 6:243, 2015; Anguille et al., Pharmacol. Rev. 67:731-753, 2015) As a result, *ex vivo* generated DCs derived from monocytes collected for the subject to be treated by, for example, leukapheresis; however, strategies using other DC types are currently being investigated. After generating DCs, the cells are generally pulsed with an antigen and infect back into the patient. The choice and source of the antigen, (*e**.**g*., purified tumor specific or tumor associated antigen

In preclinical studies, activated DC (aDC; DCVax^{®}-Direct) were shown to be superior to immature DC in clearing tumors from mice, upon intratumoral injection.

Dendritic cells (DCs) are the professional antigen presenting cells of the immune system believed to be capable of activating both naive and memory T cells. Dendritic cells are increasingly prepared *ex vivo* for use in immunotherapy, particularly for immunotherapy of cancer. The preparation of dendritic cells with optimal immunostimulatory properties requires an understanding and exploitation of the biology of these cells for *ex vivo* culture. Various protocols for the culture of these cells have been described, with various advantages ascribed to each protocol.

Activation of dendritic cells initiates the process that converts immature DCs, which are phenotypically similar to skin Langerhans cells, to mature, antigen presenting cells that can migrate to the lymph nodes. This process results in the gradual and progressive loss of the powerful antigen uptake capacity that characterizes the immature dendritic cell, and in the up-regulation of expression of co-stimulatory cell surface molecules and various cytokines. Various stimuli can initiate the maturation of DCs. This process is complex and at least *in vitro* full maturation of dendritic cells, and particularly monocytic dendritic cells, depending on the dendritic cell maturation agent used, can take up to 48 hours to complete. One other consequence of maturation is a change in the *in vivo* migratory properties of the cells. For example, the induction of immature dendritic cell maturation induces several chemokine receptors, including CCR7, which direct the cells to the T cell regions of draining lymph nodes, where the mature DCs activate T cells against the antigens presented on the DC surface in the context of class I and class II MHC molecules. The terms "activation" and "maturation", and "activated" and "mature" describe the process of inducing and completing the transition from an immature DC (partially characterized by the ability to take up antigen) to a mature DC (partially characterized by the ability to effectively stimulate *de novo* T cell responses). The terms typically are used interchangeably in the art.

Known maturation protocols are based on the *in vivo* environment that DCs are believed to encounter during or after exposure to antigens. An early example of this approach is the use of monocyte conditioned media (MCM) as a cell culture medium. MCM is generated *in vitro* by culturing monocytes and used as a source of maturation factors. (See for example, US 2002/0160430.) The major components in MCM responsible for maturation are reported to be the (pro)inflammatory cytokines Interleukin 1 beta (IL-1β), Interleukin 6 (IL-6) and tumor necrosis factor alpha (TNFα). Other dendritic cell maturation agents include, for example, Toll-like receptor agonists in mixtures of cytokines, such as tumor necrosis factor α (TNFa), interleukin (IL)-1β, IL-6 and prostaglandin E2 (PGE₂).

Maturation of DCs therefore can be triggered or initiated by a multitude of different factors that act via a host of signal transduction pathways. Consequently, there is no single maturation pathway or outcome, but there exists in fact a universe of mature DC stages, each with their own distinct functional characteristics. Conceptually this makes sense because the various threats to the body that the immune system must respond to are manifold, requiring different attack strategies. As an example, while bacterial infection is best cleared by activated macrophages supplemented with specific antibodies, a viral infection is best attacked through cytotoxic T cells that effectively kill virus-infected cells. The killing of cancer cells typically involves a combination of cytotoxic T cells, natural killer cells and antibodies.

*In vitro* maturation of DCs can therefore be designed to induce the immune system to favor one type of immune response over another, *i.e.,* to polarize the immune response. Directional maturation of DCs describes the notion that the outcome of the maturation process dictates the type of ensuing immune response that results from treatment with the matured DCs. In its simplest form, directional maturation results in a DC population that produces cytokines that direct a T cell response polarized to either a Thl-type or Th2-type immune response. Interferon γ, interferon α, and polyisosinic: polycytidylic acid have been used to supplement a dendritic cell maturation agent in order to generate mature type-1 polarized DCs that secrete IL-12. The mature DCs crease a T-helper cell 1 (T_{H}1)-type profile that elicits natural killer cell and CTL activation. (Maillard et al., Cancer Res. 64:5934-5937, 2004; Trinchieri, Blood 84:4008-4027, 1994). CTL activation triggers a pro-inflammatory state, stimulating these cells to kill tumor cells directly. (Coulie et al., Nat. Rev. Cancer 14:135-146, 2014).

DCs express up to nine different Toll-like receptors (TLR1 through TLR9), each of which can be used to trigger maturation. Not surprisingly, interaction of bacterial products with TLR2 and TLR4 results in directional maturation of DCs resulting in a polarized response most appropriate to dealing with bacterial infections. Conversely, maturation triggered through TLR7 or TLR9 appears to result more in an anti-viral type response. As an additional example, addition of interferon gamma (IFN-γ) to most maturation protocols results in the production of interleukin 12 by the mature DCs, which dictates a Thl-type immune response. Conversely, inclusion of prostaglandin E₂ has the opposite effect.

Fully mature dendritic cells differ qualitatively and quantitatively from immature DCs. Once fully mature, DCs express higher levels of MHC class I and class II antigens, and higher levels of T cell co-stimulatory molecules, such as CD80 and CD86. These changes increase the capacity of the dendritic cells to activate T cells because they increase antigen density on the cell surface, as well as the magnitude of the T cell activation signal through the counterparts of the co-stimulatory molecules on the T cells, *e.g.,* CD28 and the like. In addition, mature DCs produce large amounts of cytokines, which stimulate and polarize the T cell response. These cytokines include interleukin 12 associated with a Th1-type immune response and interleukin-10 and interleukin-4 associated with a Th2-type immune response.

Generally methods for *ex vivo* DC generation comprise obtaining a cell population enriched for DC precursor cells from a subject and then differentiating the DC precursor cells *in vitro* into fully mature DCs prior to introduction back into the subject. Typically during this process the maturing DCs are contacted with antigen for uptake and processing as the DCs become mature. Some believe that the DCs must be terminally differentiated, or they will de-differentiate back into monocytes/macrophages and lose much of their immune-potentiating ability. *Ex vivo* maturation of DCs generated from monocytes has been successfully accomplished with methods and agents well known in the art.

Dendritic cells (DCs) are recognized as the vehicle of choice for active immunotherapy of cancer. Animal experiments have demonstrated the potential of DC based immunotherapy in both protecting mice from tumor formation and eliminating established tumors. These successes have been at least partially duplicated in humans in small clinical trials. The transition from small safety- or proof-of-concept trials to larger trials in which activity or efficacy can be demonstrated has been hindered by the laborious and cumbersome nature of DC preparation as described above. As a consequence, few companies have been interested in developing DC-based cancer vaccines despite the large potential therapeutic value of such products.

In addition to maturation, the administration method has a significant impact on outcomes. The administration route must allow the DCs to reach the lymph nodes, so they can induce T cell differentiation. Several methods, including intravenous, intradermal, and intranodal injection have been studied previously. (Anguille et al., Pharmacol. Rev. 67:731-753, 2015). Intratumoral (IT) injection of DCs is a special form of DC-based immunotherapy. Upon injection, the naive DCs take up and process antigen(s) *in vivo* from, for example, apoptotic or dying (necrotic) tumor cells and tumor milieu, and present the antigen(s) to T cells after migration to the lymph nodes. Indeed, it was found that the efficacy of such treatments in animal models correlates with the degree of apoptosis in the tumor (Candido et al., Cancer Res. 61:228-236, 2001), which suggests that this approach is fully compatible with treating tumors with chemotherapeutic agents or radiation prior to the injection of DCs. In addition, several groups have demonstrated that such combination therapy is particularly effective against established tumors (Nikitina et al., Int. J. Cancer 94:825-833, 2001; Tanaka et al., Int. J. Cancer 101:265-269, 2002; Tong et al., Cancer Res. 61:7530-7535, 2001).

Since *in vivo* tumor cells are the source of antigen, intratumoral injection foregoes the need for both the selection and manufacturing of tumor antigens as they are currently used in most *in vitro* DC based therapy approaches. Selection of a tumor antigen is often driven by the need for companies to have a proprietary position and the few tumor antigens identified to date have yet to be proven to provide significant clinical benefit. In addition, the use of such tumor antigens often results in a monovalent immunogenic composition or vaccine, which can lose its effectiveness if the tumor cells down regulate the expression of the antigen used in immunization. In addition, the need to manufacture the tumor antigen under conditions required under Good Manufacturing Practices (GMP) adds additional cost to classical DC-based immunization methods.

IT injection of DCs can subject the dendritic cells to an immunosuppressive tumor environment. Tumors are known to produce cytokines that inactivate the DCs or that have the ability to skew T cell response toward a less effective Th2-type immune response. Several groups have used genetic modification of DCs to attempt to overcome these suppressive effects, especially through the production of the cytokine Interleukin 12 (IL-12; Nishioka et al., Cancer Res. 59:4035-4041, 1999; Melero et al., Gene Therapy 6:1779-1784, 1999) or the expression of CD40 ligand (Kikuchi et al., Blood 96:91-99, 2000). The encouraging results described by these groups further demonstrate the viability of IT injection of DCs as a therapeutic approach.

Triozzi et al. (Cancer 89:2647-2654, 2000) describe IT injection of DCs in patients with metastatic melanoma or breast cancer. They obtained tumor regression in 4 patients with melanoma and in two patients with breast carcinoma. Biopsies of the regressing lesions demonstrated infiltrating T cells, suggesting that the DC had indeed activated an immune response against the tumor cells. Overall these data demonstrated that IT injection of DCs was feasible in humans, and could provide significant clinical benefit. However, significant down regulation of MHC class II antigens and of the B7-2 (CD86) co-stimulatory molecule on injected DCs has been observed. Down regulation of these critical molecules would be expected to reduce the immunostimulatory potential of the DCs.

One method to overcome this down regulation has been disclosed in WO 2004/053072 where it was found that down regulation can be avoided through partial maturation of the DCs prior to administration. In this method dendritic cell precursors (bone marrow cells following red cell lysis or monocytic dendritic cell precursors) were first induced *in vitro* to differentiate into immature dendritic cells and subsequently, the immature dendritic cells were induced to begin maturation by culturing the cells with a dendritic cell maturation agent, such as BCG and IPNγ, lipopolysaccharide (LPS), tumor necrosis factor α (TNFa), an imidazoquinoline compound, a synthetic double stranded polyribonucleotide, a agonist of a Toll-like receptor (TLR), a sequence of nucleic acids containing unmethylated CpG motifs known to induce the maturation of DC, combinations of cytokines such as, for example, tumor necrosis factor α (TNFa), combined with interleukin 1β (IL-1β), interleukin 6 (IL-6), and prostaglandin E2 (PGE₂), or any combination thereof. The immature dendritic cells were allowed to continue maturation for a time period less than what had previously been determined for the immature dendritic cells to fully mature. If the dendritic cells were allowed to fully mature *in vitro* the cells would be unable to uptake and process antigen subsequent to administration to the patient. The methods disclosed herein demonstrate that the dendritic cells should be allowed to mature for a time period sufficient for activation such that significant levels of IL-6, IL-8, IL-12 and/or TNFα as set forth herein are produced prior to isolation of the partially mature dendritic cells and formulation for administration to a patient or individual in need of immunostimulation.

Unexpectedly it has been determined that activated dendritic cells which produce certain amounts, or threshold amounts, of IL-6, IL-8, IL-12 and/or TNFα have a level of immunotherapeutic potency that correlates with improved clinical outcome, measured by such characteristics as increased survival time and/or increase time to cancer recurrence. As such, activated dendritic cells which produce above the threshold amounts of IL-6, IL-8, IL-12 and/or TNFα provide improved compositions for use in administering to a subject and the compositions demonstrate an increased ability to produce a positive clinical outcome. Strioga et al (Critical Reviews in Immunology, 33(6):489-547 (2013)) describes therapeutic dendritic cell-based cancer vaccines. Jin et al (Journal of Translational Medicine 2010, 8:4) describes molecular signatures of maturing dendritic cells and implications for testing the quality of dendritic cell therapies.

### SUMMARY

The present invention is defined by the claims. This summary is provided to introduce a selection of concepts in a simplified form that are further described below in the Detailed Description. This summary is not intended to identify key features of the claimed subject matter, nor is it intended to be used as an aid in determining the scope of the claimed subject matter.

### DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:

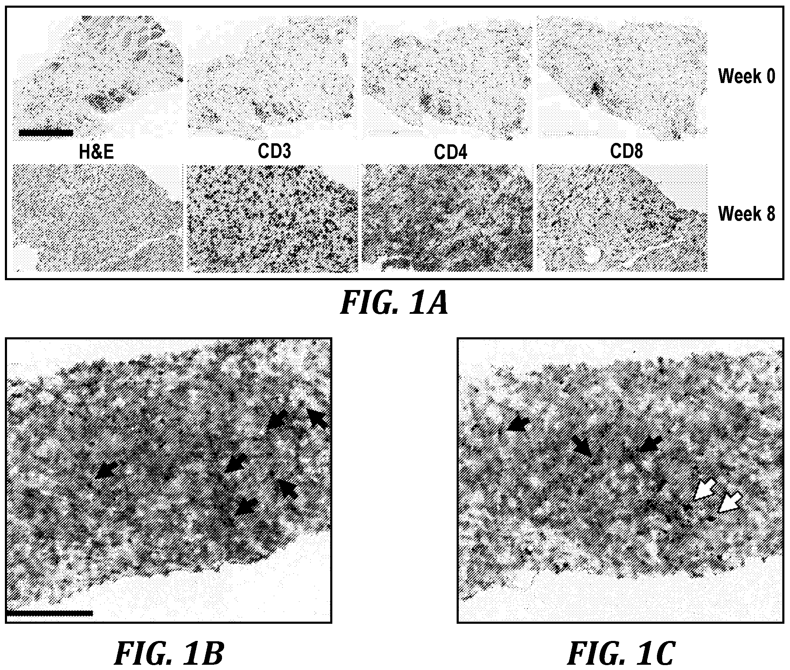

FIGURES 1A to 1C depict T cell infiltration following activated DC treatment Immunochemical staining shows that tumor infiltrating lymphocytes, including CD3⁺ activated T cells Immunohistochemical staining shows that tumor infiltrating lymphocytes, including CD3⁺ activated T cells, CD4⁺ helper cells, and CD8⁺ killer cells, increased from baseline in 15 of 27 biopsied patients Representative images are from a clear cell sarcoma tumor treated with 6 million activated DCs/ injection. Two injections had been administered at the time of biopsy. Magnification is 20×, and the scale bar represents 200 µm. Fig. 1B and Fig. 1C depicts cytokine production by activated T cells. Tissue sections were probed for: Fig. 1B IPNγ and Fig. 1C show TNFα expression using RNAscope (dark dots) and co-stained for CD3 expression (lighter dots) using immunohistochemistry. Black arrows represent CD3⁺ activated T cells expressing their respective cytokines. White arrows represent CD3⁻ cytokine-producing cells, likely macrophages. Representative images are from a clear cell sarcoma tumor treated with 6 million activated DCs/injection. Two injections had been administered at the time of biopsy. Magnification is 20× and the scale bar represents 100 µm.

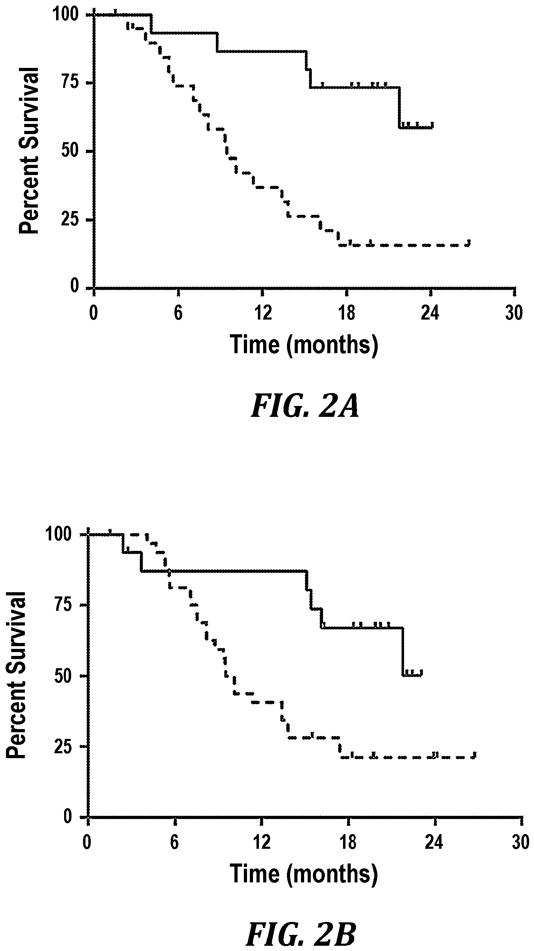

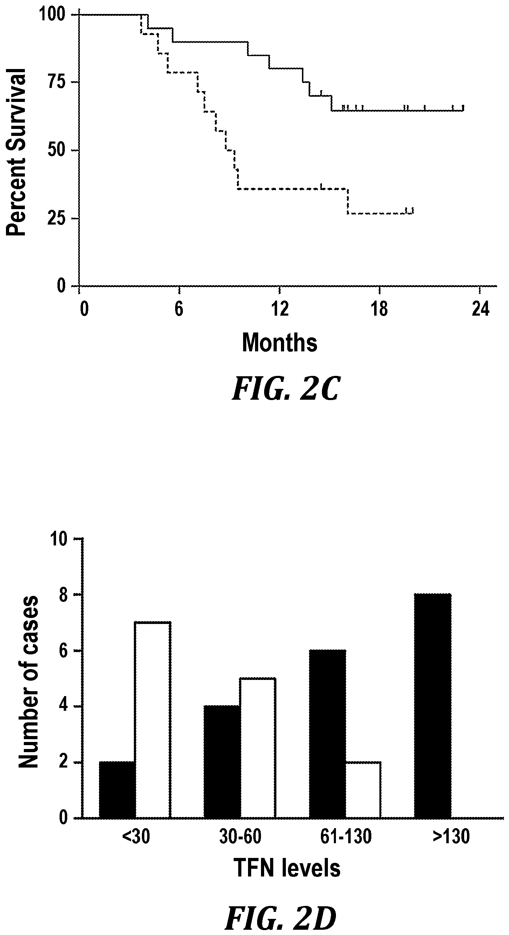

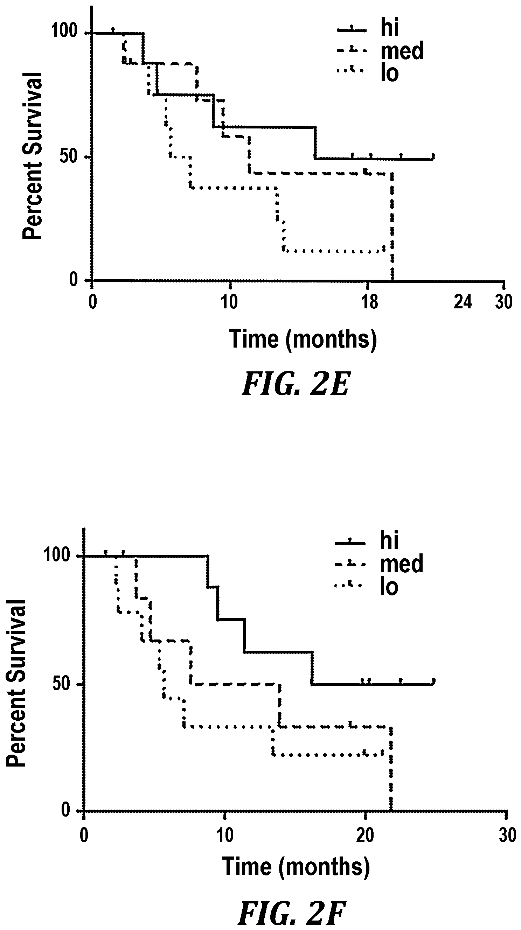

FIGURES 2A through 2F depict characterizations of activated DCs. Figure 2A shows the correlation between IL-8 production (ng/10⁶ DCs/day) and overall survival. Kaplan-Meyer curve of IL-8 production and survival. The dashed line indicates survival in patients injected with aDCs producing < 985 ng/10⁶ DCs/day (the median IL-8 concentration); the solid line represents those injected with cells producing ≥ 985 ng/10⁶ DCs/day. Figure 2B shows the correlation between IL-12p40 production (ng/10⁶ DCs/day) and overall survival. Kaplan-Meyer curve of IL-12p40 production and survival. The dashed line indicates survival in patients injected with activated DCs producing < 330 ng/10⁶ DCs/day (the median IL-12p40 concentration); the solid line represents those injected with cells producing ≥ 330 ng/10⁶ DCs/day. Figure 2C demonstrates the number of patients with stable disease (SD) at week 8 and survival. Kaplan-Meyer curve of patients with SD at week 8 compared with that of patients with progressive disease (PD) at week 8. The dashed line indicates survival in patients with PD at week 8; the solid line represents those patients with SD at week 8. The overall survival was significantly different between the two groups (p = 0.04). Figure 2D demonstrates TNFα production by the activated DCs and disease status at week 8. The number of patients with SD at week 8 is shown with black bars, and the number of patients with PD is shown with white bars. There were no patients with PD at week 8 in patients with TNFα levels > 130 ng/10⁶ DCs/day. In a multivariate analysis, TNFα production correlates with survival (p = 0.016). For Fig. 2A - Fig. 2D, n = 39. Associations between patient survival and expression levels of the cell surface markers Figure 2E measures staining for MHC-II and Figure 2F shows staining for CD86 (n = 25 in both figures). Figure 2E the solid line indicates patients with cells having > 12,000 mean fluorescence intensity (MFI) when stained for MHC-II; the dashed line indicates patients with cells having 6,200-12,000 MFI; and the dotted line represents patients with cells having < 6,200 MFI. Figure 2F the solid line indicates patients with cells having > 3,400 mean fluorescence intensity (MFI) when stained for CD86; the dashed line indicates patients with cells having 2,000 - 3,400 MFI; and the dotted line represents patients with cells having < 2,000 MFI. Log-rank analysis was used for Figure 2A - Figure 2C and Figure 2E - Figure 2F. Chi-squared analysis was used for Figure 2D.

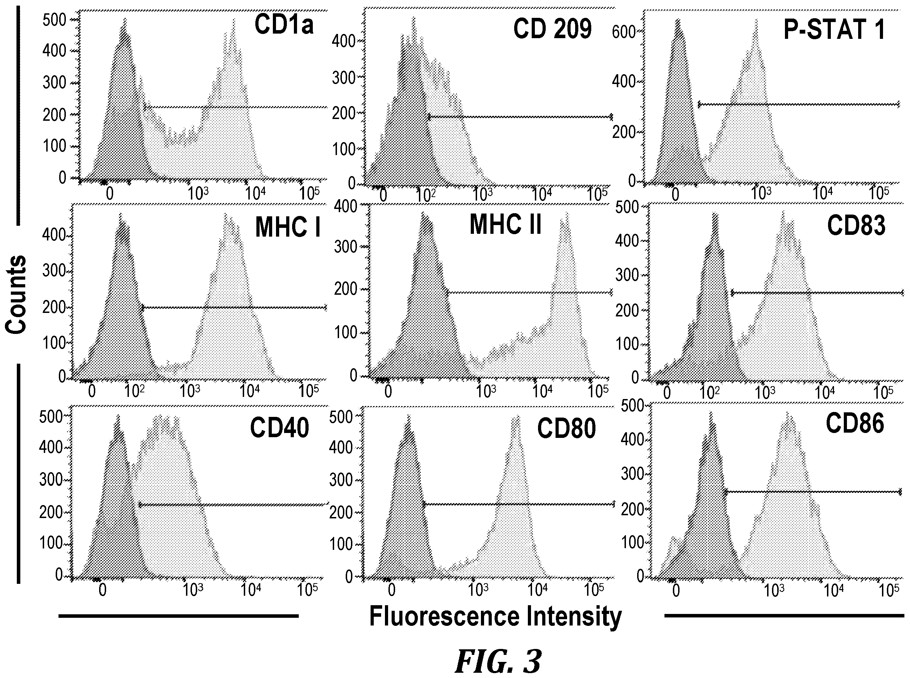

FIGURE 3 demonstrates the phenotype of activated dendritic cells. Presented are representative flow cytometry histograms of various dendritic cell-activation markers. Dark grey histograms are from the monocyte population harvested during leukapheresis. Light grey histograms are from activated DCs.

### DETAILED DESCRIPTION

The present invention is defined by the claims. The disclosure describes an *in vitro* method for determining the immunotherapeutic potency of an activated dendritic cell composition, the method comprising the steps of:
(i) preparing activated dendritic cells; (ii) determining the relative amounts of IL-6, IL-8, IL-12 and/or TNFα; (iii) comparing the determined amount of IL-6, IL-8, IL-12 and/or TNFα to a threshold amount; and (iv) determining that the activated dendritic cell composition is of low immunotherapeutic potency if one or any combination of, and/or all of IL-6, IL-8, IL-12 and/or TNFα is below threshold; or that the activated dendritic cell composition is of high immunotherapeutic potency if one or any combination of, and/or all of IL-6, IL-8, IL-12 and TNFα are above threshold.

Also described in the present disclosure is an *in vitro* method for increasing the immunotherapeutic potency of an activated DC population, the method comprises the steps of: (i) preparing an activated dendritic cell population; (ii) determining the relative amounts of IL-6, IL-8, IL-12 and/or TNFα; (iii) comparing the determined amount of IL-6, IL-8, IL-12 and/or TNFα to a threshold amount; (iv) determining whether any of one or any combination of, and/or all of IL-6, IL-8, IL-12 and/or TNFα is below threshold; and (v) adding a sufficient amount of an agent that can induce the production of one or any combination of, and or all of IL-6, IL-8, IL-12 and/or TNFα by the activated DC to bring the amount of IL-6, IL-8, IL-12 and/or TNFα to above the threshold amount so as to form an activated DC population with an increased immunotherapeutic potency.

Further, the disclosure describes an *in vitro* method for selecting a patient that will respond to administration of activated dendritic cells by determining the immunotherapeutic potency of an activated dendritic cell composition derived from the patient, the method comprising the steps of: (i) preparing activated dendritic cells; (ii) determining the relative amounts of IL-6, IL-8, IL-12 and/or TNFα; (iii) comparing the determined amount of IL-6, IL-8, IL-12 and/or TNFα to a threshold amount; and (iv) determining that the activated dendritic cell composition is of low immunotherapeutic potency if one or any combination of, and/or all of IL-6, IL-8, IL-12 and/or TNFα is below threshold, or that the activated dendritic cell composition is of high immunotherapeutic potency if one of or any combination of, and/or all of IL-6, IL-8, IL-12 and TNFα are above threshold and selecting those patients above the threshold as patients that will respond.

Still further, the disclosure describes an *in vitro* method for selecting a patient that will not respond to administration of activated dendritic cells by determining the immunotherapeutic potency of an activated dendritic cell composition derived from the patient, the method comprising the steps of: (i) preparing activated dendritic cells; (ii) determining the relative amounts of IL-6, IL-8, IL-12 and/or TNFα; (iii) comparing the determined amount of IL-6, IL-8, IL-12 and/or TNFα to a threshold amount; and (iv) determining that the activated dendritic cell composition is of low immunotherapeutic potency if one of or any combination of, and/or all of IL-6, IL-8, IL-12 and/or TNFα is below threshold, or that the activated dendritic cell composition is of high immunotherapeutic potency if one or any combination or, and/or all of IL-6, IL-8, IL-12 and TNFα are above threshold and selecting those patients below the threshold as patients that will not respond.

The disclosure further describes an *in vitro* method for selecting dendritic cell maturation agents for producing activated dendritic cells with increased immunotherapeutic potency, the method comprising the steps of: (i) preparing activated dendritic cells by contacting immature dendritic cells with a test dendritic cell maturation agent; (ii) determining the relative amounts of IL-6, IL-8, IL-12 and/or TNFα; (iii) comparing the determined amount of IL-6, IL-8, IL-12 and/or TNFα to a threshold amount; and (iv) determining that the activated dendritic cell composition is of low immunotherapeutic potency if one or any combination of, and or all of IL-6, IL-8, IL-12 and/or TNFα is below threshold, or that the activated dendritic cell composition is of high immunotherapeutic potency if one or any combination of, and/or all of IL-6, IL-8, IL-12 and/or TNFα are above threshold and selecting the dendritic cell maturation agent that induces the production of activated dendritic cells above the threshold. Once the dendritic cell maturation agent is determined it can be used to induce the production of partially mature and activated dendritic cells as described herein.

Typically, the activated dendritic cells produce threshold amounts of about 50 to about 200 ng/1 million cells/24 hours of IL-6; about 500 to about 2000 ng/1 million cells/24 hours of IL-8; at least about 30 to about 70 ng/1 million cells/24 hours of TNFα; at least about 75 to about 100 ng/1 million cells/24 hours of the IL-12 p40 subunit; and about 1 to 3 ng/1 million cells/24 hours of biologically active IL-12 p70. The activated dendritic cells can also produce about 75 to about 150 ng/1 million cells/24 hours of IL-6; about 750 to about 1500 ng/1 million cells/24 hours of IL-8; at least about 100 ng/1 million cells/24 hours of IL-12 p40; least about 1 to 3 ng/1 million cells/24 hours of IL-12 p70; and at least about 30 to 70 ng/1 million cells/24 hours of TNFα or about 100 ng/1 million cells/24 hours of IL-6, and 1000 ng/1 million cells/24 hours of IL-8, at least about 100 ng/1 million cells/24 hours of IL-12 p40; at least about 2 ng/1 million cells/24 hours of IL-12 p70; and at least about 30 ng of TNFα.

The activated dendritic cells used can be prepared by the following steps: (i) isolating a cell population comprising human PBMCs from peripheral blood; (ii) enriching the cell population comprising human PBMCs for human monocytic dendritic cell precursors; (iii) culturing the cell population enriched for human monocytic dendritic cell precursors with a tissue culture medium supplemented with an effective amount of a dendritic cell differentiation agent for a time period sufficient to differentiate the human monocytic dendritic cell precursors into immature human dendritic cells; (iv) culturing the cell population enriched for immature human dendritic cells with an effective amount of a dendritic cell maturation agent to activate the immature human dendritic cells; and (v) isolating and washing the activated human dendritic cells.

The activated dendritic cells may be prepared by the following steps: (i) isolating a cell population comprising human monocytic dendritic cell precursors; (ii) culturing the cell population enriched for human monocytic dendritic cell precursors with a tissue culture medium supplemented with an effective amount of a dendritic cell differentiation agent for a time period sufficient to differentiate the human monocytic dendritic cell precursors into immature human dendritic cells; (iii) culturing the cell population enriched for immature human dendritic cells with an effective amount of a dendritic cell maturation agent to activate the immature human dendritic cells; and (iv) isolating and washing the activated human dendritic cells. The dendritic cell differentiation agent can be GM-CSF without any other cytokine, or GM-CSF in combination with IL-4, IL-7, IL-13 or IL-15.

The monocytic dendritic cell precursors can be obtained from skin, spleen, bone marrow, thymus, lymph nodes, umbilical cord blood, or peripheral blood. In certain embodiments, the monocytic dendritic cell precursor cells are non-activated monocytic dendritic cell precursors. In addition, the monocytic dendritic cell precursors are can be obtained from the individual subject to be treated, or if the individual does not have a sufficient number of responsive monocytic dendritic cell precursors, the monocytic dendritic cell precursors can be obtained from a healthy individual subject HLA-matched to the individual subject to be treated.

Dendritic cell maturation agent useful in the methods for producing partially mature activated dendritic cells can be inactivated Bacillus Calmette-Guerin (BCG), BCG in combination with interferon γ (IPNγ), lipopolysaccharide (LPS), tumor necrosis factor α (TNFa), an imidazoquinoline compound, a synthetic double stranded polyribonucleotide, a agonist of a Toll-like receptor (TLR), a sequence of nucleic acids containing unmethylated CpG motifs known to induce the maturation of dendritic cells, or any combination thereof. The inactivated BCG can comprise whole BCG, cell wall constituents of BCG, BCG-derived lipoarabidomannans, or BCG components and the BCG can be inactivated BCG using heat-inactivation, formalin treatment, or a combination thereof. Typically, the effective amount of BCG is about 10⁵ to 10⁷ cfu per milliliter of tissue culture media and the effective amount of IFNy is about 100 to about 1,000 Units per milliliter of tissue culture media. In addition, the imidazoquinoline compound can be an imidazoquinoline-4-amine compound and typically, is 4-amino-2-ethoxymethyl-α,α-dimethyl-1H-imidazol[4,5-c]quinolin-1-5 ethanol or 1-(2-methylpropyl)-1H-imidazo[4,5-c]quinolin-4-amine, or a derivative thereof. Typically, the synthetic double stranded polyribonucleotide is poly[I]:poly[C(12)U].

Dendritic cells are a diverse population of antigen presenting cells found in a variety of lymphoid and non-lymphoid tissues. (*See* Liu, Cell 106:259-262, 2001; Steinman, Ann. Rev. Immunol. 9:271-296, 1991). Dendritic cells include lymphoid dendritic cells of the spleen, Langerhans cells of the epidermis, and veiled cells in the blood circulation. Collectively, dendritic cells are classified as a group based on their morphology, high levels of surface MHC-class II expression, and absence of certain other surface markers expressed on T cells, B cells, monocytes, and natural killer cells. In particular, monocyte-derived dendritic cells (also referred to as monocytic dendritic cells) usually express CD11c, CD80, CD86, and are HLA-DR⁺, but are CD14⁻.

In contrast, monocytic dendritic cell precursors (typically monocytes) are usually CD14⁺. Monocytic dendritic cell precursors can be obtained from any tissue where they reside, particularly lymphoid tissues such as the spleen, bone marrow, lymph nodes and thymus. Monocytic dendritic cell precursors also can be isolated from the circulatory system.

Peripheral blood is a readily accessible source of monocytic dendritic cell precursors. Umbilical cord blood is another source of monocytic dendritic cell precursors. Monocytic dendritic cell precursors can be isolated from a variety of organisms in which an immune response can be elicited. Such organisms include animals, for example, including humans, and non-human animals, such as, primates, mammals (including dogs, cats, mice, and rats), birds (including chickens), as well as transgenic species thereof.

Monocytic dendritic cell precursors and/or immature dendritic cells can be isolated from a healthy subject or from a subject in need of immunostimulation, such as, for example, a cancer patient or other subject for whom cellular immunostimulation can be beneficial or desired (*i.e.,* a subject having a bacterial or viral infection, or a hyperplastic condition, and the like). Dendritic cell precursors and/or immature dendritic cells also can be obtained from a HLA-matched healthy individual for partial activation and administration to an HLA-matched subject in need of immunostimulation. Where dendritic cell precursors and/or immature dendritic cells isolated from a subject do not form an activated dendritic cell composition that produce immunostimulatory potency factors of an appropriate level, dendritic precursor cells or immature dendritic cells from a HLA matched normal donor can be used.

### Dendritic Cell Precursors and Immature Dendritic Cells

Methods for isolating cell populations enriched for dendritic cell precursors, such as non-activated dendritic cell precursors, and immature dendritic cells from various sources, including blood and bone marrow, are known in the art. For example, dendritic cell precursors and immature dendritic cells can be isolated by collecting heparinized blood, by apheresis or leukapheresis, by preparation of buffy coats, rosetting, centrifugation, density gradient centrifugation (*e.g*., using Ficoll^{®} (such as FICOLL-PAQUE^{®}), PERCOLL^{®} (colloidal silica particles (15-30 nm diameter) coated with non-dialyzable polyvinylpyrrolidone (PVP)), sucrose, and the like), differential lysis of cells, filtration, and the like. A leukocyte population can be prepared, such as, for example, by collecting blood from a subject, de-fibrinating to remove the platelets and lysing the red blood cells. Dendritic cell precursors and immature dendritic cells can optionally be enriched for monocytic dendritic cell precursors by, for example, centrifugation through a PERCOLL^{®} gradient, antibody panning, and the like.

Dendritic cell precursors and immature dendritic cells optionally can be prepared in a closed, aseptic system. As used herein, the terms "closed, aseptic system" or "closed system" refer to a system in which exposure to non-sterile, ambient, or circulating air or other non-sterile conditions is minimized or eliminated. Closed systems for isolating dendritic cell precursors and immature dendritic cells generally exclude density gradient centrifugation in open top tubes, open air transfer of cells, culture of cells in tissue culture plates or unsealed flasks, and the like. The closed system allows aseptic transfer of the dendritic cell precursors and immature dendritic cells from an initial collection vessel to a sealable tissue culture vessel without exposure to non-sterile air.

Another reported method for isolating dendritic cell precursors is to use a commercially treated plastic substrate (*e.g.,* beads or magnetic beads) to selectively remove adherent monocytes and other "non-dendritic cell precursors." (*See, e.g.,* U.S. Patent Nos. 5,994,126 and 5,851,756). The adherent monocytes and non-dendritic cell precursors are discarded while the non-adherent cells are retained for *ex vivo* culture and maturation. In another method, apheresis cells were cultured in plastic culture bags to which plastic, *i.e.,* polystyrene or styrene, microcarrier beads were added to increase the surface area of the bag.

Cells are cultured for a sufficient period of time for certain cells to adhere to the beads and the non-adherent cells were washed from the bag. (Maffei, et al., Transfusion 40:1419-1420, 2000; WO 02/44338. ). In certain other embodiments, monocytic dendritic cell precursors are isolated by adherence to a monocyte-binding substrate, as disclosed in WO 03/010292.

For example, a population of leukocytes (*e.g.,* isolated by leukapheresis) can be contacted with a monocytic dendritic cell precursor adhering substrate. When the population of leukocytes is contacted with the substrate, the monocytic dendritic cell precursors in the leukocyte population preferentially adhere to the substrate. Other leukocytes (including other potential dendritic cell precursors) exhibit reduced binding affinity to the substrate, thereby allowing the monocytic dendritic cell precursors to be preferentially enriched on the surface of the substrate.

Suitable substrates include, for example, those having a large surface area to volume ratio. The substrate can be, for example, a particulate or fibrous substrate. Suitable particulate substrates include, for example, glass particles, plastic particles, glass-coated plastic particles, glass-coated polystyrene particles, and other beads suitable for protein absorption. Fibrous substrates suitable for use in the present invention include microcapillary tubes and microvillus membranes, and the like. The particulate or fibrous substrate typically allows the adhered monocytic dendritic cell precursors to be eluted without substantially reducing the viability of the adhered cells. A particulate or fibrous substrate can be substantially non-porous to facilitate elution of monocytic dendritic cell precursors or dendritic cells from the substrate. A "substantially non-porous" substrate is a substrate in which at least a majority of pores present in the substrate are smaller than the cells to minimize entrapping cells in the substrate.

Adherence of the monocytic dendritic cell precursors to the substrate can optionally be enhanced by the addition of a binding media. Suitable binding media include, for example, monocytic dendritic cell precursor culture media (e.g., AIM-V^{®}, RPMI 1640, DMEM, XVIVO 15^{®}, and the like) supplemented, individually or in any combination, with for example, cytokines (*e.g.,* Granulocyte/Macrophage Colony Stimulating Factor (GM-CSF), or GM-CSF in combination with Interleukin 4 (IL-4), Interleukin 15 (IL-15), or Interleukin 13 (IL-13)), blood plasma, serum (*e.g.,* human serum, such as autologous or allogeneic sera), purified proteins, such as serum albumin, divalent cations (*e.g.,* calcium and/or magnesium ions) and other molecules that aid in the specific adherence of monocytic dendritic cell precursors to the substrate, or that prevent adherence of non-monocytic dendritic cell precursors to the substrate. In certain embodiments, the blood plasma or serum can be heated-inactivated. The heat-inactivated plasma can be autologous or heterologous to the leukocytes.

Following adherence of monocytic dendritic cell precursors to the substrate, the non-adhering leukocytes are separated from the monocytic dendritic cell precursor/substrate complexes. Any suitable means can be used to separate the non-adhering cells from the complexes. For example, the mixture of the non-adhering leukocytes and the complexes can be allowed to settle, and the non-adhering leukocytes and media decanted or drained. Alternatively, the mixture can be centrifuged, and the supernatant containing the non-adhering leukocytes decanted or drained from the pelleted complexes.

In another method, non-activated monocytic dendritic cell precursors can be isolated from a cell population enriched in leukocytes prepared by the use of a tangential flow filtration device such as that described in International Patent Application Publication No., WO 2004/000444, filed June 19, 2003, now US Patent No. 7,695,627.

A tangential flow filtration device useful for the isolation of a cell population enriched in monocytic dendritic cell precursors can comprise a remover unit having a cross-flow chamber, a filtrate chamber and a filter disposed therebetween. The filter is in fluid communication on one side, the retentate surface, with the cross-flow chamber, and on the other side, the filtrate surface, with the filtrate chamber. The cross-flow chamber has an inlet adapted to introduce a sample of blood constituents comprising leukocytes into the cross-flow chamber and parallel to the retentate surface of the filter. An outlet is also provided in the cross-flow chamber centrally disposed in a portion of the chamber opposite the retentate surface of the filter. The filter suitable for use in the tangential flow filtration device typically has an average pore size ranging from about 1 to about 10 microns. The filter can have an average pore size of about 3 to about 7 microns. A means for providing a predetermined input rate of the sample into the inlet of the cross-flow chamber and a means for controlling a filtration rate of filtrate through the filter and into the filtrate chamber can also be included. The filtration rate controlling means limits the rate of filtration to less than the unopposed filtration rate for the filter. The sample comprising blood constituents can be provided by a source device such as a leukapheresis device or a container comprising a sample collected from a leukapheresis device.

Monocytic dendritic cell precursors and cell populations enriched for the precursors can be cultured *ex vivo* or *in vitro* for differentiation, and partial maturation and/or expansion. As used herein, isolated immature dendritic cells, dendritic cell precursors, and other cells, refers to cells that, by human hand, exist apart from their native environment, and are therefore not a product of nature. Isolated cells can exist in purified form, in semi-purified form, or in a non-native environment. Briefly, *in vitro* and/or *ex vivo* dendritic cell differentiation typically involves culturing monocytic dendritic cell precursors, or populations of cells having dendritic cell precursors, in the presence of one or more dendritic cell differentiation agents. Suitable differentiating agents can include, for example, cellular growth factors (*e.g*., cytokines such as (GM-CSF), or a combination of GM-CSF and Interleukin 4 (lL-4), Interleukin 13 (lL-13), Interleukin 15 (IL-15), or Interleukin 7 (IL-7)). The monocytic dendritic cells precursors may be differentiated to form monocyte-derived immature dendritic cells.

The dendritic cell precursors can be cultured and differentiated in suitable *in vitro* culture conditions. Suitable dendritic cell tissue culture media include, but are not limited to, AIM-V^{®}, RPMI 1640, DMEM, X-VIVO 15^{®}, and the like. The tissue culture media can be supplemented with serum, plasma, amino acids, vitamins, cytokines, such as GM-CSF and/or IL-4, IL-7, IL-13, IL-15, divalent cations, and the like, to promote differentiation of the cells. The dendritic cell precursors can be cultured in serum-free media. The culture conditions can optionally exclude any animal-derived products. A typical cytokine combination used with dendritic cell culture medium comprises about 500 units/ml each of GM-CSF and IL-4, IL-7, IL-15 or IL-13. In a typical embodiment where non-activated dendritic cell precursors are used a typical dendritic cell tissue culture medium can be supplemented with GM-CSF without any other cytokine. When GM-CSF is used alone the tissue culture medium is also typically supplemented with a high concentration of human or animal protein to prevent adhesion of the non-activated monocytic dendritic cell precursor to the tissue culture substrate thereby activating maturation of the dendritic cell precursor. Typically the human or animal protein is added at a concentration of greater than 1% and typically is used at a concentration of 10% or less. The human or animal protein can be an albumin, such as human serum albumin, serum, plasma, gelatin, a poly-amino acid, and the like.

Dendritic cell precursors, when differentiated to form immature dendritic cells, are phenotypically similar to skin Langerhans cells. Immature dendritic cells typically are CD14⁻ and CD11c⁺, express low levels of CD86 and CD83, and are able to capture soluble antigens via specialized endocytosis.

Dendritic cell maturation agents can include, for example, but are not limited to, BCG, LPS, TNFα, a combination of TNFα, interleukin (IL)-1β, IL-6, and prostaglandin E₂ (PGE₂), an imidazoquinoline compound, *e.g.,* a imidazoquinoline-4-amine compound, such as 4-amino-2-ethoxymethyl-α,α-dimethyl-1H-imidazol[4,5-c]quinolin-1-ethanol (designated R848) or 1-(2-methylpropyl)-1H-imidazo[4,5-c]quinolin-4-amine, and their derivatives (See for example, WO2000/47719

), a synthetic double stranded polyribonucleotide, *e.g*., poly[I]:poly[C(12)U], and the like, agonists of a Toll-like receptor (TLR), such as TLR-3, TLR-4, TLR-7 and/or TLR-9, a sequence of nucleic acids containing unmethylated CpG motifs known to induce the maturation of DC, and the like, or any combination thereof. In addition, interferon γ can be combined with one or more of the above dendritic cell maturation agents to bias the maturation of the immature dendritic cells toward a phenotype that can induce a Th1 type response. Effective amounts of BCG typically range from an equivalent to about 10⁵ to 10⁷ cfu per milliliter of tissue culture media prior to deactivation. Effective amounts of IFNy typically range from about 100 to about 1000 U per milliliter of tissue culture media.

Bacillus Calmette-Guerin (BCG) is an avirulent strain of *Mycobacterium bovis.* As used herein, BCG refers to whole BCG as well as cell wall constituents, BCG-derived lipoarabidomannans, and other BCG components. BCG is optionally inactivated, such as heat-inactivated BCG, formalin-treated BCG, or by combinations of heat and other inactivation methods, and the like. An effective amount of an imidazoquinoline compound, *e.g.,* a imidazoquinoline-4-amine compound, such as 4-amino-2-ethoxymethyl-α,α-dimethyl-1H-imidazol[4,5-c]quinolin-1-ethanol (designated R848) can be about 1 to about 50 µg/ml of culture medium, more typically 5 to about 10 µg/ml of culture media is used. The imidazoquinoline compound can be used alone or can be combined with, for example BCG and/or IPNγ), or an additional TLR agonist.

The immature DCs are typically contacted with effective amounts of the dendritic cell maturation agent, such as BCG and IPNγ, for a time period sufficient to induce maturation and to activate, but not fully mature the dendritic cells. Typically at least a 24 hour incubation period is required for complete maturation when BCG and IPNγ are used to mature dendritic cells, and depending on the dendritic cell maturation agent used, a typical incubation period of about 48 to about 72 hours is required for full maturation.

Depending on the dendritic cell maturation agent used the time period can be about 5 hours to about 19 hours, or more. More typically where BCG and IPNγ are used the time period for partial maturation and optimal activation of the dendritic cells can be about 8 to about 19 hours, or more. The immature dendritic cells can be cultured and, partially matured and activated in suitable maturation culture conditions. Suitable tissue culture media include, but are not limited to, AIM-V^{®}, RPMI 1640, DMEM, X-VIVO 15^{®}, and the like. The tissue culture media can be supplemented with amino acids; vitamins; cytokines, such as GM-CSF alone (See for example, US Patent No. 8,389,278, or GM-CSF in combination with IL-4, IL-7, IL-13, or IL-15; divalent cations; and the like, to promote the induction of maturation of the cells. A typical cytokine can be GM-CSF alone with a high concentration of human or animal protein or GM-CSF when used in combination is used at a concentration of about 500 units/ml to about 1000 units/ml of GM-CSF and 100 ng/ml of IL-4, IL-13, or IL-15 is used.

Partial maturation and activation of immature dendritic cells can be monitored by methods known in the art for dendritic cells. Cell surface markers can be detected in assays familiar to the art, such as flow cytometry, immunohistochemistry, and the like. The cells can also be monitored for cytokine production (e.g., by ELISA, another immune assay, or by use of an oligonucleotide array). In DCs cultured and partially matured and optimally activated according to the present invention in the presence of a dendritic cell maturation agent, such as for example, but not limited to, BCG and IPNγ, an increased level of phosphorylated JAK2 (janus activated kinase 2) as compared to immature dendritic cells can be measured to indicate the initiation of maturation by methods well known in the art. The induction of the expression of cell surface markers and cytokines, as well as the phosphorylation of signaling molecules, *e.g.*, jak2, is also known as an indicator that dendritic cells are conditioned for the uptake of antigen *in vivo* and the induction of an immune response once the dendritic cells have been administered to an individual.

The immature dendritic cells are subject to maturation only for a time period necessary to initiate maturation of the immature dendritic cells and to partially mature and activate the dendritic cells. Typically, a time period of about 5, or 8, or 10 to 19 hours incubation with an effective amount of BCG and an effective amount of IPNγ has been found to partially mature and activate the dendritic cells for use as a composition when combined with a pharmaceutically acceptable carrier for administration to a subject. Fully mature DCs lose the ability to take up antigen and display up-regulated expression of co-stimulatory cell surface molecules and various cytokines. Specifically, mature DCs express higher levels of MHC class I and II antigens than immature dendritic cells, and mature dendritic cells are generally identified as being CD80⁺, CD83⁺, CD86⁺, and CD14⁻. Greater MHC expression leads to an increase in antigen density on the DC surface, while up regulation of co-stimulatory molecules CD80 and CD86 strengthens the T cell activation signal through the counterparts of the co-stimulatory molecules, such as CD28 on the T cells. Partially mature and activated dendritic cells as used in the present disclosure typically comprise those dendritic cells that once exposed to a dendritic cell maturation agent demonstrate an up-regulation in the expression of a co-stimulating molecule on the cell surface as compared with immature dendritic cells. These co-stimulating molecules include, but not limited to, CD80, CD86 and/or CD54. The cells can or may not express CD83, but the cells do maintain the ability to efficiently uptake and process antigen. Further, the partially and optimally mature dendritic cells can produce one or any combination of, and/or all of TNF-α, IL-6, IL-8, IL-10 and/or IL-12 which are not typically produced in significant amounts by immature dendritic cells.

The invention involves determining the relative amounts of IL-6, IL-8, IL-12 p40, and TNF-α of activated and not fully mature human dendritic cells.

Activated dendritic cells that produce certain amounts of one or any combination of, and/or all of IL-6, IL-8, IL-12 and/or TNFα have now been correlated with improved clinical outcome. An improved clinical outcome can be measured, for example, by increased survival time and/or increased time before tumor recurrence as compared with individuals not treated or with individuals treated with a standard approved treatment protocol. In the present description it has been found that activated dendritic cells that produce about 50 to about 200 ng/1 million cells/24 hours of IL-6, about 500 to about 2000 ng/1 million cells/24 hours of IL-8; at least about 30 to about 70 ng/1 million cells/24 hours of TNFα; and/or at least about 75 to about 100 ng/1 million cells/24 hours of the IL-12 p40 subunit and about 1 to 3 ng/1 million cells/24 hours of biologically active IL-12 p70 have an immunological potency that correlates with improved clinical outcome. These cytokines/chemokines can also range between about 75 to about 150 ng/1 million cells/24 hours and preferably about 100 ng/1 million cells/24 hours of IL-6; about 750 to about 1500 ng/1 million cells/24 hours and preferably about 1000 ng/1 million cells/24 hours of IL-8; at least about 100 ng/1 million cells/24 hours of IL-12 p40 and preferably at least about 100 ng/1 million cells/24 hours of IL-12 p70 and produce an immunological potency that correlates with improved clinical outcome. As previously disclosed an improved clinical outcome is characterized by a significantly increased survival time or a significantly increased time to tumor or cancer recurrence as compared to an individual with the same cancer or tumor that is either not treated or has been treated using the currently established standard of care.

Fully mature dendritic cells are not used for the present invention because once they are fully mature the cells no longer efficiently uptake and process antigen. Further, immature dendritic cells as used in prior methods are not used, because the immunosuppressive environments typically found within a tumor, or in the tissue surrounding a tumor, include substantial concentrations of cytokines known to prevent the processing of antigen by immature dendritic cells. The present invention uses activated not fully mature human dendritic cells. In the present disclosure, partial maturation and optimal activation of the immature dendritic cells down regulates cytokine receptors on the surface of the cell rendering them less sensitive or responsive to any immunosuppressive effects of cytokines present in the intratumoral space, or surrounding tissue, and provides for cells that can efficiently uptake and process antigens present within the intratumoral space or surrounding tissue. The dendritic cells take up and process substantial amounts of tumor antigen from apoptotic and dying tumor cells found within the intratumoral space or in the surrounding tissue. Once the administered partially matured and optimally activated dendritic cells have matured within the intratumoral space as measured by, for example, the expression of the chemokine receptor CCR7, the dendritic cells migrate to the lymph nodes where the dendritic cells now presenting antigen will contact T cells to up regulate the immune response to any tumor antigens presented by the dendritic cells.

The various DCs of the disclosure can be preserved, *e.g.*, by cryopreservation as monocytic dendritic cell precursors, immature dendritic cells before maturation, or following partial maturation either in combination with or without a pharmaceutically acceptable carrier. Cryopreservation agents which can be used include but are not limited to dimethyl sulfoxide (DMSO), glycerol, polyvinylpyrrolidone, polyethylene glycol, albumin, dextran, sucrose, ethylene glycol, i-erythritol, D-ribitol, D-mannitol, D-sorbitol, inositol, D-lactose, choline chloride, amino acids, methanol, acetamide, glycerol monoacetate, and inorganic salts. A controlled slow cooling rate can be critical. Different cryoprotective agents and different cell types typically have different optimal cooling rates.

The heat of fusion phase where water turns to ice typically should be minimal. The cooling procedure can be carried out by use of, *e.g.,* a programmable freezing device or a methanol bath procedure. Programmable freezing apparatuses allow determination of optimal cooling rates and facilitate standard reproducible cooling. Programmable controlled-rate freezers such as Cryomed^{®} or Planar^{®} permit tuning of the freezing regimen to the desired cooling rate curve.

After thorough freezing, monocytic precursor cells, immature DCs and/or partially mature DCs either with or without a pharmaceutically acceptable carrier can be rapidly transferred to a long-term cryogenic storage vessel. In a typical embodiment, samples can be cryogenically stored in liquid nitrogen (-196 °C) or its vapor (-165° C). Considerations and procedures for the manipulation, cryopreservation, and long term storage of hematopoietic stem cells, particularly from bone marrow or peripheral blood, is largely applicable to the cells of the invention. Such a discussion can be found, for example, in the following references. : Taylor et al., Cryobiology 27:269-78 (1990); Gorin, Clinics in Haematology 15:19-48 (1986); Bone-Marrow Conservation, Culture and Transplantation, Proceedings of a Panel, Moscow, Jul. 2226, 1968, International Atomic Energy Agency, Vienna, pp. 107-186.

Frozen cells are preferably thawed quickly (*e.g.,* in a water bath maintained at 37 °C - 41 °C) and chilled immediately upon thawing. It may be desirable to treat the cells in order to prevent cellular clumping upon thawing. To prevent clumping, various procedures can be used, including but not limited to the addition before and/or after freezing of DNase (Spitzer et al., Cancer 45: 3075-85 (1980)), low molecular weight dextran and citrate, hydroxyethyl starch (Stiff et al., Cryobiology 20: 17-24 (1983)), and the like. The cryoprotective agent, if toxic in humans, should be removed prior to therapeutic use of the thawed partially matured DCs. One way in which to remove the cryoprotective agent is by dilution to an insignificant concentration. Once frozen monocytic dendritic cell precursors, immature dendritic cells, and/or partially matured DCs have been thawed and recovered, they can then be used in further methods to either continue with the production of partially mature activated dendritic cells or to produce a formulated pharmaceutical product. The formulated partially matured and optimally activated dendritic cells can be administered as described herein with respect to nonfrozen partially matured and optimally activated DCs.

### Determination of Immunotherapeutic Potency of the Partially Matured and Activated Dendritic Cells

The amount of various inflammatory cytokines and chemokines can be measured by methods well known in the art. In the present disclosure the amounts of one or a combination or, and/or all of IL-6, IL-8, IL-12 and/or TNFα produced by the activated dendritic cells can be associated with improved clinical outcome. Again, the invention involves determining the relative amounts of IL-6, IL-8, IL-12 p40, and TNF-α of activated and not fully mature human dendritic cells. An improved clinical outcome can be measured, for example, by a significantly increased survival time and/or a significantly increased time before tumor recurrence as compared with individuals not treated or with individuals treated with a standard approved treatment protocol. Unexpectedly it has been found that activated dendritic cells that produce about 50 to about 200 ng/1 million cells/24 hours of IL-6, about 500 to about 2000 ng/1 million cells/24 hours of IL-8; at least about 30 to about 70 ng/1 million cells/24 hours of TNFa; and/or at least about 75 to about 100 ng/1 million cells/24 hours of the IL-12 p40 subunit and about 1 to 3 ng/1 million cells/24 hours of biologically active IL-12 p70 have an immunological potency that correlates with improved clinical outcome. These cytokines/chemokines can also range between about 75 to about 150 ng/1 million cells/24 hours and preferably about 100 ng/1 million cells/24 hours of IL-6; about 750 to about 1500 ng/1 million cells/24 hours and preferably about 1000 ng/1 million cells/24 hours of IL-8; at least about 100 ng/1 million cells/24 hours of IL-12 p40 and preferably at least about 100 ng/1 million cells/24 hours of IL-12 p70.

These activated dendritic cells can be used for an immunotherapeutic potency test to determine whether an activated dendritic cell composition will likely produce an improved clinical outcome when administered back to the individual. In addition, it is described herein that the immunotherapeutic potency test can be used select patients that are likely to develop a significant immune response, reject lots of dendritic cell composition that would not be expected to produce a significant immune response when administered back to the individual; or can be used to screen dendritic cell activation agents that can produce the desired levels of one or any combination of, and/or all of IL-6, IL-8, IL-12 and/or TNFα.

It is described herein that where peripheral blood isolated from an individual does not produce activated dendritic cells capable of producing the desired levels of one or any combination of, and/or all of IL-6, IL-8, IL-12 and/or TNFα, that patient my need to be treated with activated dendritic cells isolated from an HLA-matched normal donor.

*In vitro* methods described in this disclosure for determining the immunotherapeutic potency of an activated dendritic cell composition can comprise the steps of i) preparing activated dendritic cells using any of the methods set forth above; ii) determining the relative amounts of IL-6, IL-8, IL-12 and/or TNFα using any method well known in the art; iii) comparing the determined amount of one or any combination of, and/or all of IL-6, IL-8, IL-12 and/or TNFα to a threshold amount; iv) determining that the activated dendritic cell composition is of low immunotherapeutic potency if one or any combination of, and/or all of IL-6, IL-8, IL-12 and/or TNFα is below threshold; or that the activated dendritic cell composition is of high immunotherapeutic potency if one or any combination of, and/or all of IL-6, IL-8, IL-12 and TNFα are above threshold. The threshold amounts for IL-6, IL-8, IL-12 and TNFα are set forth above. If the activated dendritic cells demonstrate high immunotherapeutic potency, the activated dendritic cells can be formulated for administration with a pharmaceutically acceptable carrier.

In another disclosure an *in vitro* method for increasing the immunotherapeutic potency of an activated DC population is described. The method comprises the steps of:
i) preparing an activated dendritic cell population; ii) determining the relative amounts of IL-6, IL-8, IL-12 and/or TNFα by a method well known in the art; iii) comparing the determined amount of one or any combination of, and/or all of IL-6, IL-8, IL-12 and/or TNFα to a threshold amount; iv) determining whether one or any combination of, and/or all of IL-6, IL-8, IL-12 and or TNFα is below threshold; v) adding a sufficient amount of an agent that can induce the production of one or any combination of, and/or all of IL-6, IL-8, IL-12 and/or TNFα by the activated DC to bring the amount of IL-6, IL-8, IL-12 and/or TNFα to above the threshold amount so as to form an activated DC population with an increased immunotherapeutic potency.

### In Vivo Administration of Partially Matured Dendritic Cells

Described herein are compositions of partially mature and activated dendritic cells, or a cell population enriched and containing such cells, for use in methods of treatment of a subject having for example, a cancer or a tumor. Such methods describd herein can be performed by obtaining dendritic cell precursors or immature dendritic cells, differentiating and partially maturing those cells in the presence of a dendritic cell maturation agent, such as BCG and IPNγ, or any other dendritic cell maturation agent such as those listed above. The partially mature and activated dendritic cells can be provided to a medical practitioner in a cryogenic state. Prior to administration, the frozen cells are quickly thawed, cooled and a formulated with a physiologically acceptable carrier, excipient, buffer and/or diluent using methods and compositions well known to the skilled artisan. The partially mature and activated dendritic cells can be administered directly to a subject in need of immunostimulation. Typically, about 10² to about 10¹⁰ cells are suspended in a pharmaceutically acceptable carrier, for example, phosphate buffered saline. The cells are injected either into the tumor directly or into a region near to, adjacent to, or in a circulatory vessel or lymphatic duct contacted with the tumor or tumor bed to ensure that the cells have access to the cancer or tumor antigen.

For example, but not by limitation, the cells can be administered directly into a tumor, into the tumor bed subsequent to surgical removal or resection of the tumor, peritumoral space, into a draining lymph node in direct contact with the tumor, into a blood vessel or lymph duct leading into, or feeding a tumor or organ afflicted by the tumor, *e.g.,* the portal vein or a pulmonary vein or artery, and the like. The administration of the partially and optimally mature dendritic cells described herein can be either simultaneous with or subsequent to other treatments for the tumor, such as chemotherapy or radiation therapy. Further, the partially mature dendritic cells described herein can be co-administered with another agent, which agent acts as an adjuvant to the maturation of the dendritic cell and/or the processing of antigen within the tumor or region near or adjacent to the tumor. In addition, the dendritic cells can also be formulated or compounded into a slow release matrix for implantation into a region in or around the tumor or tumor bed such that cells are slowly released into the tumor, or tumor bed, for contact with the tumor antigens.

A tumor as used in the present disclosure includes solid tumors, such as, for example and not limitation, a sarcoma; a pancreatic tumor; a colorectal tumor; a melanoma; a lung tumor; a breast tumor; an ovarian tumor; a head or neck tumor; a stomach tumor; a prostate tumor; an esophageal tumor; a cervical or vaginal tumor; a brain tumor, such as, for example, a glioblastoma, an astrocytoma, a meningioma, or a medulloblastoma; and the like. Additional solid tumors are also subject to treatment using a composition or method disclosed herein.

Partially mature and activated dendritic cells described herein can be administered by any means appropriate for the formulation and mode of administration. For example, the cells can be combined with a pharmaceutically acceptable carrier and administered with a syringe, a catheter, a cannula, and the like. As above, the cells can be formulated in a slow release matrix. When administered in this fashion, the formulation can be administered by a means appropriate for the matrix used. Other methods and modes of administration are well known to the skilled artisan.

Compositions des c ribe d herein can be used by themselves for use in methods of treatment of an individual described herein. In addition, the compositions can be used in combination with any other method to treat a cancer or a tumor. For example, the methods can be used in combination with surgical resection of a tumor, chemotherapy (cytotoxic drugs, apoptotic agents, antibodies, and the like), radiation therapy, cryotherapy, brachytherapy, immune therapy (administration of antigen specific mature activated dendritic cells, NK cells, antibodies specific for a cancer cell or a tumor antigen, *etc.),* and the like. Any and all of these methods can also be used in any combination. Combination treatments can be concurrent or sequential and can be administered in any order as determined by the treating physician.

In another disclosure the dendritic cells and the recipient subject have the same MHC (HLA) haplotype. Methods of determining the HLA haplotype of a subject are known in the art. In a related disclosure the partially mature dendritic cells are allogeneic to the recipient subject. The allogeneic cells are typically matched for at least one MHC allele (e.g., sharing at least one but not all MHC alleles). In a less typical disclosure the dendritic cells and the recipient subject are all allogeneic with respect to each other, but all have at least one MHC allele in common.

An anti-tumor immune response can be measured by any one or more well-known method. For example, an anti-tumor response can be measured by a reduction in the size of a tumor, the induction of tumor cell death or tumor cell necrosis, a reduction in tumor cell proliferation, or by the infiltration of tumor antigen specific T cells (TILs), and the like.

### Examples

The following example is provided merely as illustrative of various aspects of the present description and should not be construed to limit the methods and composition disclosed herein in any way.

In this example activated dendritic cells are tested in a dose escalation study in various solid tumors.

### Methods

Forty subjects were enrolled in this dose escalation study to test the safety and feasibility of intratumoral injection of activated DC (aDC), including optimally activated dendritic cells, in solid tumors. Subjects 18-75 years of age with locally advanced or metastatic disease who had undergone at least one antitumor treatment regimen within 12 weeks of screening were eligible for the study. Other eligibility criteria included having an Eastern Cooperative Oncology Group (ECOG) performance status of 0 or 1, having at least one injectable tumor mass greater than 1 cm in diameter and located away from major vascular structures or areas no amenable to swelling (*e.g.,* upper airway tumors), producing sufficient number of monocytes to manufacture a full dose course, having a life expectancy of greater than 6 months, and having adequate bone marrow and renal function. Subjects with a history of autoimmune disease or organ transplants were excluded from the study. Other exclusion criteria included having a positive status for HIV-1, 2 or HTLV-I or II; having heavily myelosuppressive or myelotoxic chemotherapy within 4 weeks prior to the first injection; receiving cancer immunotherapy within 2 years; having untreated brain metastases; needing ongoing steroid or anti-coagulant therapies; or having an acute or uncontrolled infection. Characteristics of the subjects are summarized in Table 1.

**Table 1. Baseline characteristics of treated patients**

| **Characteristics, n=39** | | **Total** |
|---|---|---|
| Age, years, median (range) | | 53 (30 - 73) |
| Sex, *n* (%) | | |
| | Male | 18(46.2) |
| | Female | 21(53.8) |
| Disease type, *n* (%) | | |
| | Pancreatic adenocancer | 5 (12.8) |
| | Sarcoma | 9 (23.1) |
| | Colorectal | 7 (17.9) |
| | Neuroendocrine | 4 (10.3) |
| | Melanoma | 6 (15.4) |
| | Lung | 3(7.7) |
| | Breast | 2(5.1) |
| | Ovarian | 1(2.6) |
| | Bladder | 1(2.6) |
| | Cholangiocarcinoma | 1(2.6) |
| No. of prior therapies, *n* (%) | | |
| | ≤ 2 | 20(51.3) |
| | 3-5 | 12 (30.8) |
| | ≥ 6 | 7 (17.9) |

### Study design

This was an evaluation of the safety and efficacy of activated DCs. The dose-escalation portion of the study used a "3 + 3" design. Three dose levels were included in this study: 2 million, 6 million, and 15 million activated DCs per injection.

Each subject underwent leukapheresis to collect monocytes, the DC precursor cells. The activated DCs (aDC; trade name DCVax^{®}-Direct) were prepared as described below. The first aDC injection took place approximately 3 weeks after the leukapheresis, and subsequent injections were administered at 1, 2, 8, 16, and 32 weeks after the first injection. All injections were administered using image guidance, either ultrasound or computed tomography (CT), to place a guide needle inside the tumor, and then a thinner needle delivered the product directly to the tumor tissue. For each immunization, 3 to 4 needle passes were used to administer the cells within the tumor margins, enhancing aDC exposure to dead and dying tumor cells while avoiding delivering a single bolus to the necrotic center of the tumor mass. After the injections, the subjects were observed for 2 hours with vital signs (heart rate, temperature, and blood pressure) taken every 30 minutes.

### Dose-limiting toxicities (DLT) and maximum tolerated dose (MTD)

DLT was defined as any of the following: ≥ grade 3 injection site reactions, development of clinical signs and symptoms of autoimmune disease, ≥ grade 2 allergic reaction, ≥ grade 2 immunological reaction that lasted for 3 or more days or required drug intervention, ≥ grade 3 National Cancer Institute Common Toxicity Criteria (NCI CTC) v.4 toxicity, or grade 4 or life-threatening events that are not related to malignancy progression. The maximum tolerated dose (MTD) was defined as the highest dose level at which no more than one third of subjects experience dose-limiting toxicities (DLT).

### Evaluation of efficacy

Treatment efficacy was evaluated by computer tomography (CT) or magnetic resonance (MR) imaging studies according to Response Evaluation Criteria in Solid Tumors v. 1.1 (Eisenhauer et al., Eur. J. Cancer 4:228-247, 2009) or immune response related criteria (Hoos et al., J. Nat'l. Cancer Inst. 102:1388-1397, 2010). Briefly, Progressing Disease (PD) was defined as a ≥ 20% increase in the sum of the target lesion's diameters compared with the smallest sum observed during the study, and the absolute sum must increase ≥ 5 mm. Stable Disease (SD) was defined as having insufficient tumor shrinkage to qualify as a partial response (≥ 30% target lesion diameter reduction), while also having insufficient tumor growth to qualify as PD.

### Preparation of activated DCs

Monocytes were purified from the leukapheresis product using tangential-flow filtration. The cells were placed in Teflon tissue culture bags (Saint-Gobain, Malvern, PA) and differentiated into immature DC for 5 days in the presence of granulocyte macrophage colony-stimulating factor (GM-CSF plus 2% human serum albumin). Cells were cultured for 5 days, and then killed BCG mycobacteria and IPNγ were added to induce DC activation for a time period of about 10 to 19 hours. Following activation, activated dendritic cells were resuspended in a small volume of RPMI-1640, 40% human serum albumin and 10% DMSO and the cells were cryopreserved in single dose aliquots. Flow cytometry was performed on the cells looking for dendritic cell-activation markers (Figure 3).

### Cytokine level determination

A custom multiplex magnetic (Luminex Corp., Austin, TX) bead set for TNFα, IL-4, IL-6, IL-8, IL-10, and IL-12p40 and a singleplex set for IL-12p70 (Invitrogen, Carlsbad, CA) were used to determine concentrations of cytokines in clarified supernatants from DCVax-Direct product cultures according to the manufacturer's protocol. Data are reported as the average value of duplicate determinations normalized per million live DCs.

### Evaluation of tumor biopsies

Biopsied tumors were formalin fixed and paraffin embedded (FFPE) using standard methods. All immunohistochemistry was performed by QualTek Molecular Laboratories (Santa Barbara, CA).

*In situ* detection of IPNγ and TNFα transcripts in FFPE specimens was performed using the RNAscope assay with probes Hs-IFNy and Hs-TNFα (cat#310501 and 310421 respectively, Advanced Cell Diagnostics (ACD), USA), as well as positive control probe PPIB (cat#313901), and RNAscope 2.0 HD Reagent kit (Brown) (cat#310035, ACD, USA) following procedures recommended by the manufacturer. To verify IPNγ and TNFα RNAscope specificity, PBMCs from three healthy donors were tested before and after T-cell stimulation. To stimulate T cells, PBMCs were isolated using Ficoll-Paque (Sigma-Aldrich), resuspended in RPMI-1640 medium supplemented with 10% fetal bovine serum and treated with 50 ng/mL phorbol myristate acetate (PMA) and 1 µg/mL ionomycin (Sigma-Aldrich) for 5 hrs at 37°C and 5% CO₂. Cells were fixed in 10% neutral buffered formalin (NBF) in Histogel^{®}, processed, and embedded into FFPE blocks. Sections (5 µm) were then tested using RNAscope as indicated above. The stimulated T cells demonstrated a strong increase in both IPNγ and TNFα compared with untreated cells. Digital images of the stained slides were acquired with an Aperio ScanScope XT digital slide scanner.

### Statistical analysis

Statistical analyses were performed to determine if cytokine levels were associated with outcome. In addition, it was assessed whether the baseline characteristics or treatment factors were predictive of the cytokine levels or outcome. Response was measured based on two variables: SD at week 8 as a binary measure and duration of survival. Adjustments were not performed for testing multiplicity. A *p* value of 0.05 was considered statistically significant.

First, descriptive measures were generated for the cytokine levels, including correlations between the potency measures. Next, the association between baseline characteristics or treatment factors with cytokine levels was assessed using non-parametric ANOVA (Wilcoxon) methods. Scatter plots of the measures were reviewed for all of the pairs of cytokine levels. A proportional hazards model was used to fit the survival as a function of the individual cytokine levels, and a backward regression was used to determine if special measures were more predictive in a joint model. A logistic model was used to fit the SD at 8 weeks as a function of the individual cytokine levels, and a backward regression was used to determine if special measures were more predictive in a joint model. Proportional hazards models, logistic models, trend tests, or likelihood-ratio χ² tests were used to evaluate the association of baseline characteristics and treatment factors with survival and SD at 8 weeks as appropriate to the measure and endpoint. For Kaplan-Meier plots of survival based on cytokine levels, the median value was used for each cytokine as the cutoff between the two groups.

Based on the analyses and a review of the scatter plots, a group of observations appeared to be potential outliers or possibly a unique set of subjects (described further in Results). The analyses were repeated with these subject records removed. Analyses were completed using SAS version 9.3 (SAS Institute Inc., Cary, NC).

### Results

### Subjects

Overall, 40 subjects were enrolled in the study. Of these, one subject was deemed not evaluable due to an incorrect formulation of the aDCs. Subject demographics and clinical characteristics are presented in Table 1. The medium subject age was 53 years old (range 30 - 73 years). The study included 21 women (53.8%). A large number of tumor types were included in the study, with the most common being sarcoma (n = 8), colorectal cancer (n = 7), and melanoma (n = 6). Subjects had a median of three lesions (range = 1 to 5 lesions). The median number of prior treatments was two (average = 3; range = 1 to 9). All procedures were done on an out-patient basis under image guidance (computed tomography) or ultrasound) facilitated by conscious sedation by an interventional radiologist. At the 2 million aDC dose, 16 subjects were administered a median of four injections (range = 1 to 6 injections). At the 6 million aDC dose, 20 subjects were administered a median of three injections (range = 2 to 6 injections). At the 15 million aDC dose, three subjects were administered a median of four injections (range = 3 to 4 injections). Only one tumor was injected per subject.

aDC were administered intratumorally under image guidance, at a dose of 2 million, 6 million, or 15 million live, activated, autologous DC per injection. At each injection visit (days 0, 7, 14, then weeks 8, 16 and 32), a single lesion was injected. To prepare the aDC for intratumoral injection, they were activated through exposure to BCG and IPNγ. Supernatants from activated DC were collected to measure cytokine production. Tumor biopsies were assessed for tumor necrosis and for infiltrating lymphocytes. Tumor size was monitored through standard imaging procedures, and blood was collected for immune monitoring.

### Safety and survival

Intratumoral injection under image guidance was generally well tolerated and feasible. In total, i.t. injections were performed, in 16 subjects at the 2 million, 20 at the 6 million, and 3 at the 15 million dose level. No dose-limiting toxicities (DLTs) were observed during the dose escalation, and thus, a maximum tolerated dose (MTD) was not determined. The maximum tested dose (15 million aDCs) was well tolerated. Adverse events related to the study treatment are reported in Table 2.

**Table 2. Treatment-related adverse events.**

| **Adverse Event^{a}** | **Activated dendritic cells (aDCs/injection)** | | | | | | |
|---|---|---|---|---|---|---|---|
| | **2 million, *n* = 16** | | **6 million, *n* = 20** | | **15 million, *n* = 3** | | **Total, *n* (%)^{b}** |
| | **G1-G2** | **G3-G4** | **G1-G2** | **G3-G4** | **G1-G2** | **G3-G4** | |
| Pyrexia | 15 | 0 | 14 | 0 | 2 | 0 | 31 (79.5) |
| Chills | 10 | 0 | 5 | 0 | 1 | 0 | 16 (41.0) |
| Fatigue | 8 | 0 | 2 | 2 | 0 | 0 | 12 (30.8) |
| Injection site | 8 | 0 | 3 | 0 | 0 | 0 | 11 (28.2) |
| pain/discomfort | | | | | | | |
| Night sweats | 5 | 0 | 5 | 0 | 0 | 0 | 10 (25.6) |
| Decreased appetite | 6 | 0 | 2 | 0 | 1 | 0 | 9 (23.1) |
| Myalgia | 4 | 0 | 3 | 0 | 0 | 0 | 7 (17.9) |
| Headache | 3 | 0 | 1 | 0 | 0 | 0 | 4 (10.3) |
| Nausea | 3 | 0 | 0 | 0 | 1 | 0 | 4 (10.3) |
| Vomiting | 3 | 0 | 0 | 0 | 1 | 0 | 4 (10.3) |
| Anemia | 1 | 0 | 0 | 1 | 0 | 0 | 2 (5.1) |
| Influenza-like illness | 1 | 0 | 1 | 0 | 0 | 0 | 2 (5.1) |
| Pain | 0 | 0 | 2 | 0 | 0 | 0 | 2 (5.1) |
| Weight loss | 0 | 0 | 1 | 0 | 1 | 0 | 2 (5.1) |
| Abdominal pain | 0 | 0 | 1 | 0 | 0 | 0 | 1 (2.6) |
| Back pain | 0 | 0 | 1 | 0 | 0 | 0 | 1(2.6) |
| Chest pain | 0 | 0 | 1 | 0 | 0 | 0 | 1 (2.6) |
| Dehydration | 1 | 0 | 0 | 0 | 0 | 0 | 1 (2.6) |
| Dry eye | 1 | 0 | 0 | 0 | 0 | 0 | 1(2.6) |
| Dry mouth | 1 | 0 | 0 | 0 | 0 | 0 | 1 (2.6) |
| Dyspnea | 0 | 0 | 1 | 0 | 0 | 0 | 1 (2.6) |
| Face edema | 0 | 0 | 1 | 0 | 0 | 0 | 1 (2.6) |
| Hydronephrosis | 1 | 0 | 0 | 0 | 0 | 0 | 1 (2.6) |
| Hypokalemia | 0 | 0 | 0 | 1 | 0 | 0 | 1 (2.6) |
| Hypomagnesaemia | 1 | 0 | 0 | 0 | 0 | 0 | 1 (2.6) |
| Insomnia | 1 | 0 | 0 | 0 | 0 | 0 | 1 (2.6) |
| Musculoskeletal | | | | | | | |
| discomfort | 1 | 0 | 0 | 0 | 0 | 0 | 1 (2.6) |
| Peripheral edema | 1 | 0 | 0 | 0 | 0 | 0 | 1 (2.6) |
| Skin sensitization | 0 | 0 | 1 | 0 | 0 | 0 | 1 (2.6) |
| Systemic inflammatory | | | | | | | |
| response syndrome | 0 | 0 | 0 | 1 | 0 | 0 | 1 (2.6) |
| Tachycardia | 0 | 0 | 1 | 0 | 0 | 0 | 1 (2.6) |

| | | | | | | | |
|---|---|---|---|---|---|---|---|
| Abbreviations: aDCs, activated dendritic cells; G, grade (according to National Cancer Institute Common Terminology Criteria for Adverse Events version 4). ^{a} When adverse events were observed on multiple dates at different grades, the highest grade observed was listed. ^{b}Percent of total patients, *N* = 39. | | | | | | | |

Treatment-related adverse events were observed in 32 subjects (82.1%), but most all of these were grade 1 or 2 and most had resolved by the end of the study period. The most common adverse events were pyrexia (n = 31, 79.5%), chills (n = 16, 41.0%), fatigue (n = 12, 23.1%), injection site pain or discomfort (n = 11, 28.2%), night sweats (n = 10, 25.6%), decreased appetite (n = 9, 23.1%), and myalgia (n = 7, 17.9%).

There were four grade 3 (10.3%) and one grade 4 (2.6%) treatment-related adverse events, all at the 6 million aDCs per injection dose.

### Histology

Serial biopsy data were collected from 28 subjects. In total, 104 biopsies were taken. New or increased necrosis was observed in 14 biopsied subjects (57%). New or increased numbers of stromal lymphocytes were observed in 14 biopsied subjects (50.0%); new or increased numbers of infiltrating lymphocytes were observed in 15 biopsied subjects (54%); and both infiltrating and stromal lymphocytes were observed in 8 biopsied subjects (29%). Biopsies were collected at week 3 and week 8, and peritumoral or intratumoral T cells were generally detected at 8 weeks post-treatment initiation. Therefore, the immune response was initiated somewhere within that timeframe. In some subjects, the T cell accumulation was detected 2 or 3 weeks after the first injection. These T cells may represent a pre-existing antitumor immune response that localizes to the tumor following activated DC injection.

*De novo* or significantly enhanced PD-L1 expression was observed in 19 of 25 evaluated tumor biopsies. Among biopsies stained for both lymphocytes and PD-L1, new or increased PD-L1 expression was observed in 9 of 12 subjects with new of increased infiltrating T cells and 11 of 12 subjects with new or increased stromal lymphocytes. Among the 19 subjects total with new or increased PD-L1 expression, 14 had either peritumoral or infiltrating lymphocytes.

When infiltrating T cells were observed, they were primarily a mixture of CD4⁺ and CD8⁺ cells; however, there were a few instances where either CD4⁺ or CD8⁺ T cells were detected exclusively. In some cases, the T cells constituted greater than 30% of total cells in the biopsy section (See also Figure 1A).

To assess tumor-associated and -infiltrating T cell functionality, RNAscope analysis was performed for IPNγ and TNFα expression on selected tissues. The majority of T cells in the samples tested were positive for both cytokines (Figures 1B and 1C), suggesting that fully functional T cells were recruited to the tumor. Tissue macrophage expressing TNFα were also detected.

### Cytokine levels, survival and stable disease (SD)

The cytokine levels of the aDCs were evaluated prior to injection in the subject. Because each batch of aDCs was derived from the subject's own monocytes, there was a large degree of inter-subject variability observed in the cytokine levels. Thus, the internal correlations between cytokine levels and the associations between cytokine levels and baseline characteristics, treatment factors, survival, and SD were evaluated at 8 weeks.

During statistical analyses, three subjects had activated DCs that had high levels of IL-8 and IL-6, but low TNFα levels. These subject consistently emerged as statistical outliers. The first outlier subject was a 51-year old male melanoma subject from the 6 million aDC treatment group. He had 5 lesions and underwent one round of treatment previously. He received three injections, had SD at week 8, and died approximately 9 months after the first injection. The second outlier subject was a 59-year old female breast cancer subject in the 6 million aDC treatment group. She had three lesions and undergone eight rounds of treatment previously. She received three injections and died approximately 1 month after the first injection. The third outlier subject was a 52-year old male lung cancer subject in the 15 million aDC treatment group. He had three lesions and underwent five rounds of treatment previously. He received three injections, had PD (Progressive Disease) at week 8, and died approximately 3.5 months after the first injection. All three subjects had a heavy burden of disease and an extremely poor prognosis. The three subjects did not have other known prominent features that separated them from the rest of the study subjects. An exploratory analysis of the aDCs from one of the subjects suggests that the purified monocytes may have failed to completely transform into aDCs. In the subsequent analyses, the outlier subject data have been excluded.

### Internal correlations between cytokine levels

To assess the quality of activation and the effect of the cytokines produced by the aDCs, the levels of TNFα, IL-6, IL-8, IL-10, IL12p40, and IL-12p70 were determined. There was a high level of internal correlation between the various cytokines evaluated. Those values were correlated with outcome (stable disease (SD) or survival) in univariate analyses. Separately, a backward regression model was used to assess the relative predictive strength of the measures and identify variable combinations based on a joint model, starting with all of the factors. The correlated cytokines were sorted into three groups. The first group included IL-6, IL-12p40and to a lesser extent TNFα. The Pearson r value for IL-6 and IL-12p40 was 0.64 (p = 0.004). The r value for IL-6 and TNFα was 0.88 (p = < 0.001). The r value for IL-8 and IL-12p40 was 0.641 (p < 0.001). The second group was IL-10 and IL-8. The r value for IL-18 and IL-10 was 0.63 (p < 0.001). The third group was IL-12p40 and IL-12p70, which had an r value of 0.55 (p < 0.001). It should be noted that the short activation time used to generate aDCs was not optimal to detect the full complement of EL-12p70 production.

### Association between cytokine levels and baseline characteristics and treatment factors

Next, associations between cytokine level and baseline characteristics and treatment factors, including indications, number of lesions, prior treatment, dose, number of injections, age, sum or the longest tumor diameter (SLD), and absolute lymphocyte count at screening (ALC) was determined using regression analysis. SLD was negatively associated with levels of IL-8 (R² = 0.20; p = 0.006), IL-12p40 (R2 = 0.14; p = 0.026) and IL-12p70 (R2 = 0.11; p = 0.051), and positively associated with IL-10 levels (R2 = 0.023). ALC was positively associated with IL-12p40 (R2 = 0.26; p = 0.002). Neither SLD or ALC were independently associated with survival.

### Associations between cytokine levels and survival

The cytokine levels were individually fit in a proportional hazard model to determine whether they were predictive of survival. Univariate analysis indicated the IL-6 (p = 0.048), IL-8 (p = 0.014), and IL-12p40 (p = 0.016) were associated with survival. Specifically, IL-8 levels greater than 985 ng/10⁶ cells/day and IL-12p40 levels greater than 330 /106 cells/day showed significantly higher overall survival (p = 0.0022 and p = 0.0077, respectively; Figures 2A and 2B).

An exploratory analysis was performed to evaluate a joint model of isolated cytokine pairs and assess potential factor interactions. The combination of IL-8 and IL-12p40 was associated with a potentially significant interaction term (p = 0.020). The joint interation model indicates that subjects with high values of both IL-8 and IL-12p40 may respond better overall. This result suggests that there may be more complex relationships between DC potency measures and clinical outcomes.

### Association between cytokine level and Stable Disease at week 8

Kaplan-Meyer analysis showed that survival was significantly associated with Week 8 SD (p = 0.004), Figure 2C); thus, it was determined whether there were cytokine markers associated with SD. The cytokine levels were individually fit into a logistic model to determine whether they were predictive of Week 8 SD. Univariate analysis revealed a positive association between Week 8 SD and TNFα (p = 0.015, Figure 2D), and this association was confirmed in a multivariate backward regression model (p = 0.014).

### Other measures of DC quality

The injected aDC from 25 subjects were analyzed for surface marker expression. Weak correlations between survival and the levels of expression (mean fluorescence intensity divided into tertiles) of MHC class I antigens (log-rank p for trend = 0.07) and the CD86 costimulatory molecule (log-rank p for trend = 0.1), lending further support for the hypothesis that DC quality is a primary driver fir subject outcome when delivered intratumorally (Figure 2E and Figure 2F).

Stabilization of disease was observed in more subjects treated with aDC that produced high levels of TNF (p < 0.01). Survival is likewise associated with high production levels of TNFα, IL-6 and IL-8.

In this study, safety and efficacy of activated autologous dendritic cells (aDCs) were tested. The aDCs were injected intratumorally, as a treatment for subjects with unresectable, locally advanced, or metastatic solid tumors. Subjects were treated with 2, 6, or 15 million aDCs per injection at week 0, 1, 2, 8, 16 and 32, or until there were no more autologous aDCs to administer. No DLTs were observed, and thus, there was no MTD. This observation is consistent with other DC vaccine studies in which no DLTs or MTDs were identified (Butterfield, Front. Immunol. 4:454, 2013, Draube et al., PLoS One 6:e18801, 2011). Given that DC vaccines use autologous cells, it was not surprising that there was limited toxicity. It has been noted previously that a DC vaccine dose is limited solely by the number of cells that can be extracted during leukapheresis and converted for treatment, which is referred to as the maximum feasible dose (Anguille et al., Pharmacol. Rev. 67:731-753, 2015).

The maximum dose administered herein was 15 million aDCs per injection, and this dose was well tolerated; however, this large dose may not be necessary to generate an effective T-cell response. One large meta-analysis of renal and prostate cancer DC vaccine trials identified a positive correlation between dose and outcome (Draube et al., PLoS One 6:e18801, 2011). However, several other studies have shown that fewer cells can achieve equivalent immune responses with relatively few DCs, as long as the DCs effectively reach the draining lymph (Tel et al., Cancer Res. 73:1063-1075, 2013; Aarntzen et al., Clin. Cancer Res. 19:1525-1533, 2013, Verdijk et al., Expert Opin. Biol. Ther. 87:865-874, 2008), Celli et al., Blood 120:3945-3948, 2012). Relatively few low-grade, treatment-related adverse events were seen with aDC treatment in this study. These adverse events were primarily associated with activation of the immune system, such as pyrexia. These results, coupled with the lack of MTD, indicate that aDCs are a safe treatment for solid tumors.

With respect to the efficacy of the aDCs, biopsies of injected tumors showed increased necrosis and infiltration of lymphocytes, including CD4⁺ helper cells and CD8⁺ killer cells. In individual cases, immune reactivity was observed with both rapid and delayed infiltration of T cells in patient biopsies and extensive necrosis. These observations preceded a demonstrable reduction in tumor size (data not shown). Studies have shown that increased infiltration and accumulation of certain types of T cells, such as stromal lymphocytes and CTLs, in tumors is strongly correlated with improved outcomes in several solid tumors (Tosolini et al., Cancer Res. 71:1263-1271, 2011; Smyth et al., Adv. Immunol. 90:1-50, 2006; Clemente et al., Cancer 77:1303-1310, 1996). In addition, PD-L1 was upregulated in 19 of 25 tumors tested, and this upregulation likely reflects the tumor response to immune activation, particularly because tumor biopsies that tested positive for T cells were more likely to have increased PD-L1 expression. PD-L1 is a co-inhibitory molecule elicited during lymphocyte infiltration that downregulates T-cell activity to control excessive immune reactions, and tumors use it to evade immune responses (Ito et al., Biomed. Res. Int. 2015:605478, 2015, Anguille et al., Pharmacol. Rev. 67:731-753, 2015). Given that the above PD-L1 data are from biopsied tumors, the emergence of PD-L1 expression may serve as a marker of successful antitumor immune response induction, rather than an indication of immune response downregulation. Overall, these results provide evidence that aDCs stimulate an effective T-cell response in solid tumors.

For the aDC treatment to be effective, it should also improve patient outcomes. It is hypothesized herein that the survival mechanism was related to DC potency, as measured by the cytokines secreted by the aDCs. Therefore, cytokine levels of the aDCs were assessed prior to injecting them into the tumors. It was observed that IL-12p40 was significantly associated with survival. IL-12p40 is one subunit of the heterodimeric IL-12 complex, also called IL-12p70. IL-12 is known to stimulate natural killer cells and mature T cells. It is also known to help convert T_{H}2 cells to T_{H}1 cells that have antitumor activity (Del Vecchio et al., Clin. Cancer Res. 12:4677-4685, 2007). Thus, IL-12p40-producing aDCs are ideal for an effective DC vaccine. In addition, IL-8 secretion was associated with survival. Specifically, high IL-8 secretion showed significantly higher overall survival. IL-8 is largely considered to be negatively associated with cancer. IL-8 promotes angiogenesis, cell proliferation, and cell survival; however, it also promotes infiltration of immune cells into the tumor microenvironment (Waugh and Wilson, Clin. Cancer Res. 14:6735-6741, 2008). In the case of BCG immunotherapy, IL-8 was associated with the development of an antitumor immune response (de Boer et al., Urol. Res. 25:31-34, 1997). It seems possible that the localized application of IL-8-producing aDCs stimulated infiltration of immune cells into the tumor. In addition, the regression model indicated that the combination of the two was positively associated with survival. This observation indicates that the combination of IL-8 and IL-12p40 (and possibly other cytokines), rather than individual cytokines, can be key for improved survival. Currently, it is unclear whether the secreted cytokines shown to correlate with survival have direct functional significance or whether they are serving as sensitive measures of overall DC potency. The observed associations between patient baseline parameters and DC potency as measured by cytokine production suggests that factors, such as SLD and ALC, may predispose patients towards greater benefit from DC-based therapies, although the R² values suggest that these baseline parameters only explain up to 25% of the variation in cytokine levels. This possibility deserves further attention in subsequent studies with more homogenous patient populations, and will be the subject of future investigations.

Additional survival analysis of the aDC-treated patients showed that SD at week 8 was significantly correlated with survival. These data indicate that if the tumor can be stabilized by aDCs, then the odds of progression-free survival significantly increase. Therefore, it was investigated what cytokines were associated with Week 8 SD. Analysis of the cytokine levels showed that TNFα was positively associated with Week 8 SD. TNFα is a well-characterized cytokine extensively associated with upregulating the immune response, including DC maturation and T-cell priming, proliferation, and recruitment (Calzascia et al., J. Clin. Invest. 117:3833-3845, 2007; van Horssen et al., Oncologist 11:397-408, 2006). Human studies have shown that isolated limb perfusion of TNFα can be used to treat locally advanced soft-tissue sarcomas (Eggermont et al., Lancet Oncol. 4:429-437, 2006). In addition, TNFα has been shown to be critical for antitumor immune responses in mice (Calzascia et al., J. Clin. Invest. 117:3833-3845, 2007). The observed positive association herein between TNFα is consistent with these results. A significant internal correlation was also observed between IL-6, IL-8, and IL-12p40; however, the apparent significance could be the result of an internal correlation between IL-8 and IL-12p40 rather than a biologically relevant one. Alternatively, it could be a reflection of overall DC potency, as discussed above. This hypothesis is supported by the correlative trends between survival and expression of MHC-II and CD86, critical DC maturation markers, on the surface of the aDCs (Steinman and Banchereau, Nature 449:419-426, 2007).

It has been shown herein that activated DCs (aDCs) are a safe, feasible treatment option for patients with solid tumors. Specific cytokines have been identified that, when one or more are secreted by the aDCs, lead to stabilization of disease, resulting in prolonged survival. Given the data above, it is clear that aDCs are a promising treatment to extend the survival of patients with unresectable, locally advanced, or metastatic solid tumors without significant toxicity in multiple solid tumors, and can elicit local and systemic immune responses. Clinical outcomes such as stabilization of disease and survival are significantly associated with DC potency measures such as cytokine production *in vitro.*

Based on the results herein it has been found that (i) intratumoral (i.t.) injection of activated dendritic cells is safe and well tolerated; (ii) clinical outcomes following i.t. injection of activated DC are correlated with DC potency, measured by cytokine production; (iii) individual cytokines show different associations with clinical outcome parameters, suggesting complex correlations between DC function and possible therapeutic benefit.

## Claims

1. An *in vitro* method for determining the immunotherapeutic potency of an activated not fully mature human dendritic cell composition for use in treating a solid tumor, the method comprising the steps of:
i) preparing activated dendritic cells that have not fully matured as measured by retaining the ability to efficiently uptake and process antigen;
ii) determining the relative amounts of Interleukin 6 (IL-6), Interleukin 8 (IL-8), Interleukin 12 p40 (IL-12 p40), and Tumor Necrosis Factor α (TNFα) of the activated and not fully mature dendritic cell composition;
iii) comparing the determined amount of IL-6, IL-8, IL-12 p40, and TNFα to a threshold amount; and
iv) determining that the activated and not fully mature human dendritic cell composition is of low immunotherapeutic potency if all of IL-6, IL-8, IL-12 p40 and TNFα are below threshold; or that the activated and not fully mature human dendritic cell composition is of high immunotherapeutic potency if all of IL-6, IL-8, IL-12 p40 and TNFα are above threshold.

2. An *in vitro* method for selecting a patient having a solid tumor that will respond or not respond to administration of activated not fully mature human dendritic cells by determining the immunotherapeutic potency of an activated and not fully mature human dendritic cell composition derived from the patient, the method comprising the steps of:
i) preparing a composition comprising activated and not fully mature human dendritic cells as measured by retaining the ability to efficiently uptake and process antigen;
ii) determining the relative amounts of IL-6, IL-8, IL-12 p40, and TNFα of the activated and not fully mature human dendritic cells;
iii) comparing the determined amount of IL-6, IL-8, IL-12 p40, and TNFα to a threshold amount; and
iv) determining that the activated and not fully mature human dendritic cell composition is of low immunotherapeutic potency if all of IL-6, IL-8, IL-12 p40, and TNFα are below threshold, or that the activated and not fully mature human dendritic cell composition is of high immunotherapeutic potency if all of IL-6, IL-8, IL-12 p40, and TNFα are above threshold and selecting those patients above the threshold as patients that will respond or selecting those patients below the threshold as patients that will not respond.

3. An *in vitro* method for selecting a dendritic cell maturation agent for producing activated and not fully mature human dendritic cells with increased immunotherapeutic potency for use in treating a solid tumor, the method comprising the steps of:
i) preparing activated and not fully mature human dendritic cells by contacting immature dendritic cells with a test dendritic cell maturation agent for a time period sufficient to induce maturation of the immature dendritic cells but not allowing the dendritic cells to fully mature as determined by the ability of the dendritic cells to efficiently uptake and process antigen;
ii) determining the relative amounts of IL-6, IL-8, IL-12 p40, and TNFα;
iii) comparing the determined amount of IL-6, IL-8, IL-12 p40, and TNFα to a threshold amount; and
iv) determining that the activated and not fully mature human dendritic cell composition is of low immunotherapeutic potency if all of IL-6, IL-8, IL-12 p40, and TNFα are below threshold, or that the activated and not fully mature dendritic cell composition is of high immunotherapeutic potency if all of IL-6, IL-8, IL-12 p40, and TNFα are above threshold and selecting the dendritic cell maturation agent that induces the *in vitro* production of the activated and not fully mature dendritic cell composition above the threshold.

4. The *in vitro* method of any one of claims 1 through 3, wherein the activated and not fully mature human dendritic cells produce 50 to 200 ng/1 million cells/24 hours of IL-6; 500 to 2000 ng/1 million cells/24 hours of IL-8; and/or at least 75 to 100 ng/1 million cells/24 hours of the IL-12 p40 subunit.

5. The *in vitro* method of claim 4, wherein the human activated not fully mature dendritic cells produce 75 to 150 ng/1 million cells/24 hours of IL-6; 750 to 1500 ng/1 million cells/24 hours of IL-8; and/or at least 100 ng/1 million cells/24 hours of IL-12 p40; preferably
wherein the activated and not fully mature dendritic cells produce 100 ng/1 million cells/24 hours of IL-6, and 1000 ng/1 million cells/24 hours of IL-8, and/or at least 100 ng/1 million cells/24 hours of IL-12 p40.

6. The *in vitro* method according to any one of claims 1 through 5, wherein the activated and not fully mature human dendritic cells are prepared by the following steps:
i) isolating a cell population comprising human peripheral blood mononuclear cells (PBMCs) from peripheral blood;
ii) enriching the cell population comprising human PBMCs for human monocytic dendritic cell precursors;
iii) culturing the cell population enriched for human monocytic dendritic cell precursors with a tissue culture medium supplemented with an effective amount of a dendritic cell differentiation agent for a time period sufficient to differentiate the human monocytic dendritic cell precursors into immature human dendritic cells;
iv) culturing the cell population enriched for immature human dendritic cells with an effective amount of a dendritic cell maturation agent to activate and not fully mature the immature human dendritic cells as determined by the efficiency of the human dendritic cells to uptake and process antigen; and
v) isolating and washing the activated and not fully mature human dendritic cells.

7. The *in vitro* method according to any one of claims 1 through 5, wherein the activated and not fully mature human dendritic cells are prepared by the following steps:
i) isolating a cell population enriched for and comprising human monocytic dendritic cell precursors;
ii) culturing the cell population enriched for human monocytic dendritic cell precursors with a tissue culture medium supplemented with an effective amount of a dendritic cell differentiation agent for a time period sufficient to differentiate the human monocytic dendritic cell precursors into immature human dendritic cells;
iii) culturing the cell population enriched for immature human dendritic cells with an effective amount of a dendritic cell maturation agent to activate and induce the maturation of the immature human dendritic cells; and
iv) isolating and washing the activated and not fully mature human dendritic cells.

8. The *in vitro* method according to claim 6 or 7, wherein the human monocytic dendritic cell precursors are obtained from skin, spleen, bone marrow, thymus, lymph nodes, umbilical cord blood, or peripheral blood.

9. The *in vitro* method according to any one of claims 6 to 8, wherein the human monocytic dendritic cell precursor cells are non-activated human monocytic dendritic cell precursors.

10. The *in vitro* method according to any one of claims 6 to 9, wherein the human monocytic dendritic cell precursors are obtained from the individual subject to be treated for the solid tumor; or wherein the human monocytic dendritic cell precursors are obtained from a healthy individual subject HLA-matched to the individual subject to be treated for the solid tumor.

11. The *in vitro* method according to claim 6 or 7, wherein the human dendritic cell differentiation agent is GM-CSF without any other cytokine, or GM-CSF in combination with IL-4, IL-7, IL-13 or IL-15.

12. The *in vitro* method according to claim 6 or 7, wherein the human dendritic cell maturation agent is inactivated Bacillus Calmette-Guerin (BCG), interferon γ (IPNγ), lipopolysaccharide (LPS), tumor necrosis factor α (TNFa), an imidazoquinoline compound, a synthetic double stranded polyribonucleotide, a agonist of a Toll-like receptor (TLR), a sequence of nucleic acids containing unmethylated CpG motifs known to induce the maturation of dendritic cells, or any combination thereof; preferably
wherein the maturation agent is a combination of BCG and IPNγ and the synthetic double stranded polyribonucleotide is poly[I]:poly[C(12)U].

13. The *in vitro* method according to claim 12, wherein the inactivated BCG comprises whole BCG, cell wall constituents of BCG, BCG-derived lipoarabidomannans, or BCG components; preferably
wherein the inactivated BCG is heat-inactivated BCG, formalin-treated BCG, or heat-inactivated and formalin treated BCG.

14. The *in vitro* method according to any one of claims 12 or 13, wherein the effective amount of BCG is 10⁵ to 10⁷ cfu per milliliter of tissue culture media and the effective amount of IFNγ is 100 to 1,000 Units per milliliter of tissue culture media.

15. The *in vitro* method according to claim 12, wherein the imidazoquinoline compound is an imidazoquinoline-4-amine compound; preferably
wherein the imidazoquinoline-4-amine compound is 4-amino-2-ethoxymethyl-α,α-dimethyl-1H-imidazol[4,5-c]quinolin-1-5 ethanol or 1-(2-methylpropyl)-1H-imidazo[4,5-c]quinolin-4-amine, or a derivative thereof.

## Patentansprüche

1. In-vitro-Verfahren zum Bestimmen der immuntherapeutischen Wirksamkeit einer aktivierten, nicht völlig ausgereiften humanen dendritischen Zellzusammensetzung zur Verwendung bei der Behandlung eines soliden Tumors, wobei das Verfahren die folgenden Schritte beinhaltet:
i) Herstellen von aktivierten dendritischen Zellen, die nicht völlig ausgereift sind, gemessen an der Beibehaltung der Fähigkeit, Antigen effizient aufzunehmen und zu verarbeiten;
ii) Bestimmen der relativen Mengen an Interleukin 6 (IL-6), Interleukin 8 (IL-8), Interleukin 12 p40 (IL-12 p40) und Tumornekrosefaktor α (TNFα) der aktivierten und nicht völlig ausgereiften dendritischen Zellzusammensetzung;
iii) Vergleichen der bestimmten Menge an IL-6, IL-8, IL-12 p40 und TNFα mit einer Schwellenmenge; und
iv) Feststellen, dass die aktivierte und nicht völlig ausgereifte humane dendritische Zellzusammensetzung von geringer immuntherapeutischer Wirksamkeit ist, wenn alle von IL-6, IL-8, IL-12 p40 und TNFα unter dem Schwellenwert liegen; oder dass die aktivierte und nicht völlig ausgereifte humane dendritische Zellzusammensetzung von hoher immuntherapeutischer Wirksamkeit ist, wenn alle von IL-6, IL-8, IL-12 p40 und TNFα über dem Schwellenwert liegen.

2. In-vitro-Verfahren zum Auswählen eines Patienten mit einem soliden Tumor, der auf die Verabreichung von aktivierten, nicht völlig ausgereiften humanen dendritischen Zellen anspricht oder nicht anspricht, durch Bestimmen der immuntherapeutischen Wirksamkeit einer aktivierten und nicht völlig ausgereiften humanen dendritischen Zellzusammensetzung, die von dem Patienten stammt, wobei das Verfahren die folgenden Schritte beinhaltet:
i) Herstellen einer Zusammensetzung, die aktivierte und nicht völlig ausgereifte humane dendritische Zellen umfasst, gemessen an der Beibehaltung der Fähigkeit, Antigen effizient aufzunehmen und zu verarbeiten;
ii) Bestimmen der relativen Mengen an IL-6, IL-8, IL-12 p40 und TNFα der aktivierten und nicht völlig ausgereiften humanen dendritischen Zellen;
iii) Vergleichen der bestimmten Menge an IL-6, IL-8, IL-12 p40 und TNFα mit einer Schwellenmenge; und
iv) Feststellen, dass die aktivierte und nicht völlig ausgereifte humane dendritische Zellzusammensetzung von niedriger immuntherapeutischer Wirksamkeit ist, wenn alle von IL-6, IL-8, IL-12 p40 und TNFα unter dem Schwellenwert liegen, oder dass die aktivierte und nicht völlig ausgereifte humane dendritische Zellzusammensetzung von hoher immuntherapeutischen Wirksamkeit ist, wenn alle von IL-6, IL-8, IL-12 p40 und TNFα über dem Schwellenwert liegen, und Auswählen der Patienten über dem Schwellenwert als Patienten, die ansprechen werden, oder Auswählen der Patienten unter dem Schwellenwert als Patienten, die nicht ansprechen werden.

3. In-vitro-Verfahren zum Auswählen eines Mittels zur Reifung dendritischer Zellen zum Produzieren aktivierter und nicht völlig ausgereifter humaner dendritischer Zellen mit erhöhter immuntherapeutischer Wirksamkeit zur Verwendung bei der Behandlung eines soliden Tumors, wobei das Verfahren die folgenden Schritte beinhaltet:
i) Herstellen aktivierter und nicht völlig ausgereifter humaner dendritischer Zellen durch Inkontaktbringen unreifer dendritischer Zellen mit einem Testmittel zur Reifung dendritischer Zellen für einen Zeitraum, der ausreicht, um die Reifung der unreifen dendritischen Zellen zu induzieren, der es aber nicht zulässt, dass die dendritischen Zellen völlig ausreifen, wie an der Fähigkeit der dendritischen Zellen bestimmt, Antigen effizient aufzunehmen und zu verarbeiten;
ii) Bestimmen der relativen Mengen an IL-6, IL-8, IL-12 p40 und TNFα;
iii) Vergleichen der bestimmten Menge an IL-6, IL-8, IL-12 p40 und TNFα mit einer Schwellenmenge; und
iv) Feststellen, dass die aktivierte und nicht völlig ausgereifte humane dendritische Zellzusammensetzung von niedriger immuntherapeutischer Wirksamkeit ist, wenn alle von IL-6, IL-8, IL-12 p40 und TNFα unter dem Schwellenwert liegen, oder dass die aktivierte und nicht völlig ausgereifte dendritische Zellzusammensetzung von hoher immuntherapeutischer Wirksamkeit ist, wenn alle von IL-6, IL-8, IL-12 p40 und TNFα über dem Schwellenwert liegen, und Auswählen des Reifungsmittels für dendritische Zellen, das die *In*-*vitro*-Produktion der aktivierten und nicht völlig ausgereiften dendritischen Zellzusammensetzung über dem Schwellenwert induziert.

4. In-vitro-Verfahren nach einem der Ansprüche 1 bis 3, wobei die aktivierten und nicht völlig ausgereiften humanen dendritischen Zellen 50 bis 200 ng/1 Mio. Zellen/24 Stunden IL-6; 500 bis 2000 ng/1 Mio. Zellen/24 Stunden IL-8; und/oder mindestens 75 bis 100 ng/1 Mio. Zellen/24 Stunden der IL-12 p40-Untereinheit produzieren.

5. In-vitro-Verfahren nach Anspruch 4, wobei die aktivierten, nicht völlig ausgereiften humanen dendritischen Zellen 75 bis 150 ng/1 Mio. Zellen/24 Stunden IL-6; 750 bis 1500 ng/1 Mio. Zellen/24 Stunden IL-8; und/oder mindestens 100 ng/1 Mio. Zellen/24 Stunden IL-12 p40 produzieren; wobei vorzugsweise
die aktivierten und nicht völlig ausgereiften dendritischen Zellen 100 ng/1 Mio. Zellen/24 Stunden IL-6, und 1000 ng/1 Mio. Zellen/24 Stunden IL-8 und/oder mindestens 100 ng/1 Mio. Zellen/24 Stunden IL-12 p40 produzieren.

6. In-vitro-Verfahren nach einem der Ansprüche 1 bis 5, wobei die aktivierten und nicht völlig ausgereiften humanen dendritischen Zellen durch die folgenden Schritte hergestellt werden:
i) Isolieren einer Zellpopulation, die humane mononukleäre Zellen des peripheren Blutes (PBMCs) umfasst, aus peripherem Blut;
ii) Anreichern der Zellpopulation, die PBMCs umfasst, mit Vorläufern humaner monozytärer dendritischer Zellen;
iii) Kultivieren der mit humanen monozytischen dendritischen Zellvorläufern angereicherten Zellpopulation mit einem Gewebekulturmedium, das mit einer wirksamen Menge eines Mittels zur Differenzierung dendritischer Zellen ergänzt ist, für einen Zeitraum, der ausreicht, um die humanen monozytischen dendritischen Zellvorläufer in unreife humane dendritische Zellen zu differenzieren;
iv) Kultivieren der mit unreifen humanen dendritischen Zellen angereicherten Zellpopulation mit einer wirksamen Menge eines Mittels zur Reifung dendritischer Zellen, um die unreifen humanen dendritischen Zellen zu aktivieren und nicht völlig auszureifen, wie anhand der Effizienz der humanen dendritischen Zellen bestimmt, Antigen aufzunehmen und zu verarbeiten; und
v) Isolieren und Waschen der aktivierten und nicht völlig ausgereiften humanen dendritischen Zellen.

7. In-vitro-Verfahren nach einem der Ansprüche 1 bis 5, wobei die aktivierten und nicht völlig ausgereiften humanen dendritischen Zellen durch die folgenden Schritte hergestellt werden:
i) Isolieren einer Zellpopulation, die mit humanen monozytären dendritischen Zellvorläufern angereichert ist und diese umfasst;
ii) Kultivieren der mit humanen monozytären dendritischen Zellvorläufern angereicherten Zellpopulation mit einem Gewebekulturmedium, das mit einer wirksamen Menge eines Mittels zur Differenzierung dendritischer Zellen ergänzt ist, für einen Zeitraum, der ausreicht, um die humanen monozytären dendritischen Zellvorläufer in unreife humane dendritische Zellen zu differenzieren;
iii) Kultivieren der mit unreifen humanen dendritischen Zellen angereicherten Zellpopulation mit einer wirksamen Menge eines Mittels zur Reifung dendritischer Zellen, um die Reifung der unreifen humanen dendritischen Zellen zu aktivieren und zu induzieren; und
iv) Isolieren und Waschen der aktivierten und nicht völlig ausgereiften humanen dendritischen Zellen.

8. In-vitro-Verfahren nach Anspruch 6 oder 7, wobei die Vorläufer humaner monozytärer dendritischer Zellen aus Haut, Milz, Knochenmark, Thymus, Lymphknoten, Nabelschnurblut oder peripherem Blut gewonnen werden.

9. In-vitro-Verfahren nach einem der Ansprüche 6 bis 8, wobei die Vorläuferzellen humaner monozytärer dendritischer Zellen nicht aktivierte Vorläuferzellen humaner monozytärer dendritischer Zellen sind.

10. In-vitro-Verfahren nach einem der Ansprüche 6 bis 9, wobei die humanen monozytischen dendritischen Zellvorläufer aus dem individuellen Subjekt gewonnen werden, das wegen des soliden Tumors behandelt werden soll; oder wobei die humanen monozytischen dendritischen Zellvorläufer aus einem gesunden individuellen Subjekt mit HLA-Übereinstimmung mit dem individuellen Subjekt gewonnen werden, das wegen des soliden Tumors behandelt werden soll.

11. In-vitro-Verfahren nach Anspruch 6 oder 7, wobei das Mittel zur Differenzierung humaner dendritischer Zellen GM-CSF ohne ein anderes Zytokin oder GM-CSF in Kombination mit IL-4, IL-7, IL-13 oder IL-15 ist.

12. In-vitro-Verfahren nach Anspruch 6 oder 7, wobei das Mittel zur Reifung humaner dendritischer Zellen inaktiviertes B-Acillus Calmette-Guerin (BCG), Interferon γ (IFNy), Lipopolysaccharid (LPS), Tumornekrosefaktor α (TNFα), eine Imidazochinolinverbindung, ein synthetisches doppelsträngiges Polyribonukleotid, ein Agonist eines Tolllike-Rezeptors (TLR), eine Nukleinsäuresequenz, die unmethylierte CpG-Motive enthält, von denen bekannt ist, dass sie die Reifung dendritischer Zellen induzieren, oder eine beliebige Kombination davon; wobei vorzugsweise
das Reifungsmittel eine Kombination aus BCG und IFNγ ist und das synthetische doppelsträngige Polyribonukleotid poly[I]:poly[C(12)U] ist.

13. In-vitro-Verfahren nach Anspruch 12, wobei das inaktivierte BCG ganzes BCG, Zellwandbestandteile von BCG, BCG-abgeleitete Lipoarabidomannane oder BCG-Komponenten umfasst; wobei vorzugsweise
das inaktivierte BCG hitzeinaktiviertes BCG, formalinbehandeltes BCG oder hitzeinaktiviertes und formalinbehandeltes BCG ist.

14. In-vitro-Verfahren nach Anspruch 12 oder 13, wobei die wirksame Menge an BCG 10⁵ bis 10⁷ cfu pro Milliliter Gewebekulturmedium und die wirksame Menge an IFNγ 100 bis 1000 Einheiten pro Milliliter Gewebekulturmedium beträgt.

15. In-vitro-Verfahren nach Anspruch 12, wobei die Imidazochinolinverbindung eine Imidazochinolin-4-Amin-Verbindung ist, wobei vorzugsweise
die Imidazochinolin-4-Amin-Verbindung 4-Amino-2-ethoxymethyl-α,α-Dimethyl-1H-imidazol[4,5-c]chinolin-1-5-ethanol oder 1-(2-Methylpropyl)-1-H-imidazo[4,5-c]chinolin-4-amin oder ein Derivat davon ist.

## Revendications

1. Procédé in vitro destiné à déterminer la puissance immunothérapeutique d'une composition de cellules dendritiques humaines pas complètement matures activées pour une utilisation dans le traitement d'une tumeur solide, le procédé comprenant les étapes suivantes :
i) préparation de cellules dendritiques activées qui ne sont pas complètement matures comme est mesuré par la conservation de la capacité à capter et à traiter efficacement un antigène ;
ii) détermination des quantités relatives d'Interleukine 6 (IL-6), d'Interleukine 8 (IL-8), d'Interleukine 12 p40 (IL-12 p40), et de facteur de nécrose tumorale α (TNFα) de la composition de cellules dendritiques activées et pas complètement matures ;
iii) comparaison de la quantité déterminée d'IL-6, d'IL-8, d'IL-12 p40, et de TNFα avec une quantité seuil ; et
iv) détermination que la composition de cellules dendritiques humaines activées et pas complètement matures présente une puissance immunothérapeutique faible si tous les IL-6, IL-8, IL-12 p40 et TNFα sont au-dessous d'un seuil ; ou que la composition de cellules dendritiques humaines activées et pas complètement matures présente une puissance immunothérapeutique élevée si tous les IL-6, IL-8, IL-12 p40 et TNFα sont au-dessus d'un seuil.

2. Procédé in vitro destiné à sélectionner un patient ayant une tumeur solide qui répondra ou ne répondra pas à l'administration de cellules dendritiques humaines pas complètement matures activées par la détermination de la puissance immunothérapeutique d'une composition de cellules dendritiques humaines activées et pas complètement matures dérivée du patient, le procédé comprenant les étapes suivantes :
i) préparation d'une composition comprenant des cellules dendritiques humaines activées et pas complètement matures comme est mesuré par la conservation de la capacité à capter et à traiter efficacement un antigène ;
ii) détermination des quantités relatives d'IL-6, d'IL-8, d'IL-12 p40, et de TNFα des cellules dendritiques humaines activées et pas complètement matures ;
iii) comparaison de la quantité déterminée d'IL-6, d'IL-8, d'IL-12 p40, et de TNFα avec une quantité seuil ; et
iv) détermination que la composition de cellules dendritiques humaines activées et pas complètement matures présente une puissance immunothérapeutique faible si tous les IL-6, IL-8, IL-12 p40 et TNFα sont au-dessous d'un seuil ; ou que la composition de cellules dendritiques humaines activées et pas complètement matures présente une puissance immunothérapeutique élevée si tous les IL-6, IL-8, IL-12 p40 et TNFα sont au-dessus d'un seuil et la sélection de ces patients au-dessus du seuil comme des patients qui répondront ou la sélection de ces patients au-dessous du seuil comme des patients qui ne répondront pas.

3. Procédé in vitro destiné à sélectionner un agent de maturation de cellules dendritiques destiné à produire des cellules dendritiques humaines activées et pas complètement matures avec une puissance immunothérapeutique augmentée pour une utilisation dans le traitement d'une tumeur solide, le procédé comprenant les étapes suivantes :
i) préparation de cellules dendritiques humaines activées et pas complètement matures par mise en contact de cellules dendritiques immatures avec un agent de maturation de cellules dendritiques d'essai pendant une période de temps suffisante pour provoquer la maturation des cellules dendritiques immatures mais sans laisser les cellules dendritiques mûrir complètement comme est déterminé par la capacité des cellules dendritiques à capter et à traiter efficacement un antigène ;
ii) détermination des quantités relatives d'IL-6, d'IL-8, d'IL-12 p40, et de TNFα ;
iii) comparaison de la quantité déterminée d'IL-6, d'IL-8, d'IL-12 p40, et de TNFα avec une quantité seuil ; et
iv) détermination que la composition de cellules dendritiques humaines activées et pas complètement matures présente une puissance immunothérapeutique faible si tous les IL-6, IL-8, IL-12 p40 et TNFα sont au-dessous d'un seuil, ou que la composition de cellules dendritiques activées et pas complètement matures présente une puissance immunothérapeutique élevée si tous les IL-6, IL-8, IL-12 p40 et TNFα sont au-dessus d'un seuil et la sélection de l'agent de maturation de cellules dendritiques qui provoque la production in vitro de la composition de cellules dendritiques activées mais pas complètement matures au-dessus du seuil.

4. Procédé in vitro selon l'une quelconque des revendications 1 à 3, dans lequel les cellules dendritiques humaines activées et pas complètement matures produisent de 50 à 200 ng/1 million de cellules/24 heures d'IL-6 ; de 500 à 2 000 ng/1 million de cellules/24 heures d'IL-8 ; et/ou au moins 75 à 100 ng/1 million de cellules/24 heures de la sous-unité IL-12 p40.

5. Procédé in vitro selon la revendication 4, dans lequel les cellules dendritiques pas complètement matures activées humaines produisent de 75 à 150 ng/1 million de cellules/24 heures d'IL-6 ; de 750 à 1 500 ng/1 million de cellules/24 heures d'IL-8 ; et/ou au moins 100 ng/1 million de cellules/24 heures d'IL-12 p40 ; préférablement
dans lequel les cellules dendritiques matures activées et pas complètement matures produisent 100 ng/1 million de cellules/24 heures d'IL-6, et 1 000 ng/1 million de cellules/24 heures d'IL-8, et/ou au moins 100 ng/1 million de cellules/24 heures d'IL-12 p40.

6. Procédé in vitro selon l'une quelconque des revendications 1 à 5, dans lequel les cellules dendritiques humaines activées et pas complètement matures sont préparées par les étapes suivantes :
i) isolement d'une population de cellules comprenant des cellules mononucléaires de sang périphérique humaines (PBMC) de sang périphérique ;
ii) enrichissement de la population de cellules comprenant des PBMC humaines en précurseurs de cellules dendritiques monocytaires humaines ;
iii) culture de la population de cellules enrichie en précurseurs de cellules dendritiques monocytaires humaines avec un milieu de culture tissulaire additionné d'une quantité efficace d'un agent de différenciation de cellules dendritiques pendant une période de temps suffisante pour différencier les précurseurs de cellules dendritiques monocytaires humaines en cellules dendritiques humaines immatures ;
iv) culture de la population de cellules enrichie en cellules dendritiques humaines immatures avec une quantité efficace d'un agent de maturation de cellules dendritiques pour activer les cellules dendritiques humaines immatures et provoquer la maturation non complète de celles-ci comme est déterminé par l'efficacité des cellules dendritiques humaines à capter et à traiter un antigène ; et
v) isolement et lavage des cellules dendritiques humaines activées et pas complètement matures.

7. Procédé in vitro selon l'une quelconque des revendications 1 à 5, dans lequel les cellules dendritiques humaines activées et pas complètement matures sont préparées par les étapes suivantes :
i) isolement d'une population de cellules enrichie en et comprenant des précurseurs de cellules dendritiques monocytaires humaines ;
ii) culture de la population de cellules enrichie en précurseurs de cellules dendritiques monocytaires humaines avec un milieu de culture tissulaire additionné d'une quantité efficace d'un agent de différenciation de cellules dendritiques pendant une période de temps suffisante pour différencier les précurseurs de cellules dendritiques monocytaires humaines en cellules dendritiques humaines immatures ;
iii) culture de la population de cellules enrichie en cellules dendritiques humaines immatures avec une quantité efficace d'un agent de maturation de cellules dendritiques pour activer et provoquer la maturation des cellules dendritiques humaines immatures ; et
iv) isolement et lavage des cellules dendritiques humaines activées et pas complètement matures.

8. Procédé in vitro selon la revendication 6 ou 7, dans lequel les précurseurs de cellules dendritiques monocytaires humaines sont obtenus à partir de la peau, de la rate, de la moelle osseuse, du thymus, de nœuds lymphoïdes, de sang de cordon ombilical, ou de sang périphérique.

9. Procédé in vitro selon l'une quelconque des revendications 6 à 8, dans lequel les cellules de précurseur de cellules dendritiques monocytaires humaines sont des précurseurs de cellules dendritiques monocytaires humaines non activées.

10. Procédé in vitro selon l'une quelconque des revendications 6 à 9, dans lequel les précurseurs de cellules dendritiques monocytaires humaines sont obtenus à partir du sujet individuel devant être traité pour la tumeur solide ; ou dans lequel les précurseurs de cellules dendritiques monocytaires humaines sont obtenus à partir d'un sujet individuel sain au typage HLA compatible avec le sujet individuel devant être traité pour la tumeur solide.

11. Procédé in vitro selon la revendication 6 ou 7, dans lequel l'agent de différenciation de cellules dendritiques humaines est un GM-CSF sans autre cytokine, ou un GM-CSF en combinaison avec une IL-4, une IL-7, une IL-13 ou une IL-15.

12. Procédé in vitro selon la revendication 6 ou 7, dans lequel l'agent de maturation de cellules dendritiques humaines est un Bacille Calmette-Guérin (BCG) inactivé, un interféron γ (IFNγ), un lipopolysaccharide (LPS), un facteur de nécrose tumorale α (TNFα), un composé imidazoquinoline, un polyribonucléotide double brin synthétique, un agoniste d'un récepteur de type Toll (TLR), une séquence d'acides nucléiques contenant des motifs CpG non méthylés connus pour provoquer la maturation des cellules dendritiques, ou toute combinaison de ceux-ci ; préférablement
dans lequel l'agent de maturation est une combinaison de BCG et d'IFNγ et le polyribonucléotide double brin synthétique est un poly[I] : poly[C(12)U].

13. Procédé in vitro selon la revendication 12, dans lequel le BCG inactivé comprend un BCG complet, des constituants de paroi de cellule de BCG, des lipoarabidomannans dérivé du BCG, ou des composants de BCG ; préférablement
dans lequel le BCG inactivé est un BCG inactivé par la chaleur, un BCG traité à la formaline, ou un BCG inactivé par la chaleur et traité à la formaline.

14. Procédé in vitro selon l'une quelconque des revendications 12 ou 13, dans lequel la quantité efficace de BCG est de 10⁵ à 10⁷ cfu par millilitre de milieu de culture tissulaire et la quantité efficace d'IFNy est de 100 à 1 000 unités par millilitre de milieu de culture tissulaire.

15. Procédé in vitro selon la revendication 12, dans lequel le composé imidazoquinoline est un composé imidazoquinoline-4-amine ; préférablement
dans lequel le composé imidazoquinoline-4-amine est le 4-amino-2-éthoxyméthyl-α,α-diméthyl-1H-imidazol[4,5-c] quinolin-1-5 éthanol ou la 1-(2-méthylpropyl)-1H-imidazo[4,5-c]quinolin-4-amine, ou un dérivé de ceux-ci.
